# EUROPEAN PATENT SPECIFICATION

(11) **EP 3 107 371 B1**
(45) Date of publication and mention of the grant of the patent: **13.01.2021**
(21) Application number: 15705605.2
(22) Date of filing: 18.02.2015
(51) Int. Cl.: A01H 3/00, A01H 3/04, C12N 15/82

(54) **METHOD FOR OBTAINING PLANTS WITH INTERMEDIATE HEIGHT**
METHODE ZUR HERSTELLUNG VON PFLANZEN VON MITTLERER HÖHE
PROCÉDÉ D'OBTENTION DE PLANTES PRÉSENTANT UNE HAUTEUR INTERMÉDIAIRE

(30) Priority: 18.02.2014 NL 2012284
(43) Date of publication of application: 28.12.2016
(73) Proprietor: Knud Jepsen A/S, 8382 Hinnerup (DK)
(72) Inventor: NIELSEN, Kai Lønne, DK-8382 Hinnerup (DK)
(74) Representative: Wittop Koning, Tom Hugo
(86) International application number: PCT/EP2015/053438
(87) International publication number: WO 2015/124640

(56) References cited:
- EP-A1- 0 838 150
- EP-A1- 2 698 432
- WO-A2-03/029444
- US-A- 5 648 598
- KIM YOUNG SEON ET AL: "Improvement of ornamental characteristics in Rehmannia elata through Agrobacterium rhizogenes-mediated transformation", PLANT OMICS, vol. 5, no. 4, July 2012 (2012-07), pages 376-380, XP002730760,
- CHRISTENSEN AND R MÜLLER B: "The Use of Agrobacterium rhizogenes and its rol-Genes for Quality Improvement in Ornamentals", EUROPEAN JOURNAL OF HORTICULTURAL SCIENCE, VERLAG EUGEN ULMER, STUTTGART, DE, vol. 74, no. 6, 1 January 2009 (2009-01-01), pages 275-287, XP007922284, ISSN: 1611-4426 cited in the application
- BRIAN CHRISTENSEN ET AL: "Transformation of Kalanchoe blossfeldiana with rol-genes is useful in molecular breeding towards compact growth", PLANT CELL REPORTS, SPRINGER, BERLIN, DE, vol. 27, no. 9, 3 July 2008 (2008-07-03), pages 1485-1495, XP019626152, ISSN: 1432-203X cited in the application
- GAURI SAXENA ET AL: "Rose-scented geranium (Pelargonium sp.) generated by Agrobacterium rhizogenes mediated Ri-insertion for improved essential oil quality", PLANT CELL, TISSUE AND ORGAN CULTURE, KLUWER ACADEMIC PUBLISHERS, DO, vol. 90, no. 2, 7 July 2007 (2007-07-07), pages 215-223, XP019534298, ISSN: 1573-5044, DOI: 10.1007/S11240-007-9261-0
- OTANI M ET AL: "Transformation of sweet potato (Ipomoea batatas (L.) Lam.) plants by Agrobacterium rhizogenes", PLANT SCIENCE, ELSEVIER IRELAND LTD, IE, vol. 94, no. 1-2, 1 January 1993 (1993-01-01), pages 151-159, XP025220259, ISSN: 0168-9452, DOI: 10.1016/0168-9452(93)90016-S [retrieved on 1993-01-01]
- HENRIK LÜTKEN ET AL: "Inheritance of-genes fromthrough two generations in", EUPHYTICA, KLUWER ACADEMIC PUBLISHERS, DO, vol. 188, no. 3, 29 April 2012 (2012-04-29), pages 397-407, XP035140328, ISSN: 1573-5060, DOI: 10.1007/S10681-012-0701-5
- WANG B ET AL: "Genetic transformation of Echinacea purpurea with Agrobacterium rhizogenes and bioactive ingredient analysis in transformed cultures", COLLOIDS AND SURFACES. B, BIOINTERFACES, ELSEVIER, AMSTERDAM, NL, vol. 53, no. 1, 1 November 2006 (2006-11-01), pages 101-104, XP027996459, ISSN: 0927-7765 [retrieved on 2006-11-01]

## Description

The present invention relates to a method for obtaining a plant having a height of less than the height of a corresponding wild type plant.

Compact plant architecture is more and more desired in the field of ornamental plants. The potted plant industry favours more compact plant architecture for environmental, space and transportation purposes. Potted plants displaying an elongated growth behaviour are losing interest of both the breeders, plant producers and the consumers.

At present, the industry treats plants with growth retardants to limit plant size. However, use of growth retardants may be harmful to both human health and the environment. However, for plants having an elongated growth habit, such as e.g. *Kalanchoe, roses and Chrysanthemum,* use of chemical growth retardants is necessary to obtain compact plants. Another disadvantage is that the use of such growth retardants results in belated and less flowering, which is a major drawback for growers of ornamental plants.
Plants transformed with *Agrobacterium rhizogenes* having compact plant architecture are described (Christensen and Müller, 2009, Europ. J. Hort. Sci. 74(6) 275-287). In that study, several plant species have been transformed by infection with *Agrobacterium rhizogenes,* and transformants were visually evaluated. The said transformants displayed numerous phenotypes, in particular dwarfism and compact plants growth. However, said compact growth often coincides with undesired phenotypes, such as wrinkled leaves, flower shape, reduced flower number, delayed flowering. For ornamental plants, the disadvantages of the *Agrobacterium rhizogenes* transfection appeared to be larger than the envisaged advantages of compact growth, in particular because the growth is retarded to an undesired extent, leading to dwarfism.

For example a *Kalanchoe blossfeldiana* transformed with *Agrobacterium rhizogenes* is described, a horticulturally important succulent plant displaying an elongated growth habit in nature and having turgid and succulent leaves that enable the plants to survive drought conditions, explaining its popularity as both an indoor and outdoor plant. The transformant displayed undesired dwarfism growth behaviour, and in addition significant disadvantages such as wrinkled leaves, a reduced number of flowers and flower size were observed. The same is true for other plans, tested in this study. Therefore, *Agrobacterium rhizogenes* transformation never gained commercial importance.

The Ri-plasmid of naturally occurring soil bacterium A. *rhizogenes* agropine-type strains carry two T-DNA regions (T_{L}-DNA and T_{R}-DNA, SEQ ID NO 1 and 2, respectively) on the Ri-plasmid for transfer into plant cells. Following infection of a plant cell, the bacterium transfers the entire T-DNA region (both T_{L}-DNA and T_{R}-DNA), thereby transferring *rol* (root oncogenic loci) and *aux* genes into the plant genome and causing hairy root growth at the site of infection (Tepfer (1984) Cell, 37, pp. 959-967). Because *A. rhizogenes* naturally infects plants, the *rol* genes are naturally transferred into the plant and function as plant oncogenes and develop hairy roots in plant tissues.

The T_{L}-DNA contains four *rol* genes, *rol*A*, rol*B*, rol*C*,* and *rol*D (SEQ ID Nos 3 - 6, respectively), whereas the T_{R}-DNA contains several genes, including two auxin genes, *aux1* and *aux2* (SEQ ID Nos 7 and 8, respectively). T-DNA comprises in total 18 open reading frames (Veena and Taylor (2007), In Vitro Cellular & Developmental Biology - Plant, 43, 383-403).

### TERMINOLOGY

All technical terms used herein are terms commonly used in biochemistry, molecular biology and agriculture, and can be understood by one of ordinary skill in the art. Technical terms can be found in: Molecular Cloning: A Laboratory Manual, 3rd ed., vol. 1-3, ed. Sambrook and Russell, Cold Spring Harbor Laboratory Press, Cold Spring Harbor, N.Y., 2001; Current Protocols in Molecular Biology, ed. Ausubel et al., Greene Publishing Associates and Wiley-Interscience, New York, 1988 (with periodic updates); Short Protocols in Molecular Biology: A Compendium of Methods from Current Protocols in Molecular Biology, 5th ed., vol. 1-2, ed. Ausubel et al., John Wiley & Sons, Inc., 2002; Genome Analysis: A Laboratory Manual, vol. 1-2, ed. Green et al., Cold Spring Harbor Laboratory Press, Cold Spring Harbor, N.Y., 1997. Methodology involving plant biology techniques is described herein and is described in detail in treatises such as Methods in Plant Molecular Biology: A Laboratory Course Manual, ed. Maliga et al., Cold Spring Harbor Laboratory Press, Cold Spring Harbor, N.Y., 1995. Various techniques using PCR are described in Innis et al., PCR Protocols: A Guide to Methods and Applications, Academic Press, San Diego, 1990 and in Dieffenbach and Dveksler, PCR Primer: A Laboratory Manual, 2nd ed., Cold Spring Harbor Laboratory Press, Cold Spring Harbor, N.Y., 2003. PCR-primer pairs can be derived from known sequences by known techniques such as using computer programs intended for that purpose, Primer, Version 0.5, 1991, Whitehead Institute for Biomedical Research, Cambridge, MA. Methods for chemical synthesis of nucleic acids are discussed, for example, in Beaucage and Caruthers, 1981, Tetra. Letts. 22: 1859-1862, and Matteucci and Caruthers, 1981 J. Am. Chem. Soc. 103: 3185. Restriction enzyme digestions, phosphorylations, ligations and transformations were done as described in Sambrook et al., Molecular Cloning: A Laboratory Manual, 2nd ed. (1989), Cold Spring Harbor Laboratory Press. All reagents and materials used for the growth and maintenance of bacterial cells were obtained from Aldrich Chemicals (Milwaukee, WI), DIFCO Laboratories (Detroit, MI), Invitrogen (Gaithersburg, MD), or Sigma Chemical Company (St. Louis, MO), unless otherwise specified.

"Transformation" refers herein to any methodology for introducing one or more genes originating from a Ri plasmid of *Agrobacterium rhizogenes* into a host plant cell. In particular, said one or more genes comprise one or more *rol* genes. Importantly, and because *A. rhizogenes* naturally infects plants, transformation includes the natural transfer of wild-type genes from the Ri plasmid from the wild-type bacterium into a plant cell, in particular *rol* genes. Thus, and as used herein, transformation neither implies nor requires cloning a heterologous gene into a vector for transfer into a host plant cell. Furthermore, a host plant cell expressing one or more genes originating from a Ri plasmid of *Agrobacterium rhizogenes,* such as one or more *rol* genes may be characterized as "transformed." Transformation may occur by any known method including, for example, natural infection, floral dip, infiltration, or particle bombardment. Transformation of a cell may be detected by any known means, including but not limited to Northern Blot, Southern blot, PCR, RT-PCR (syn. q-PCR, qRT-PCR) and/or genomic sequencing. It is also possible to take one or more genes or open reading frames, originating from the Ri plasmid of *Agrobacterium tumefaciens* from the said plasmid, and use it outside the context of the *Agrobacterium tumefaciens* system to transform an envisaged plant. The skilled person will be aware of such methods.

The term "tissue culture" refers to plant tissues propagated under sterile conditions, often for producing clones of a plant. Plant tissue culture relies on the fact that many plant cells have the ability to regenerate a whole plant. Single cells, plant cells without cell walls (protoplasts), pieces of leaves, or roots can often be used to generate a new plant on culture media given the required nutrients and plant hormones.

As used herein, "interspecific hybrid" includes the progeny from the cross of two different species of plants of the same genus and its cultivars, as well as progeny resulting from subsequent backcrossing to one of the parents. This backcrossing to one of the parents may be conducted one or more times with the goal of stably combining the double-type trait with desired characteristics. "Interspecific hybrid" embraces any plant with an interspecific cross in its background. That is, interspecific hybrids include both the first and subsequent generations of crosses between two plant species, as well as the progeny produced from either selfing an interspecific hybrid or crossing an interspecific hybrid with a plant of the same or different species.

*A. rhizogenes* refers to *Agrobacterium rhizogenes* and its Ri-plasmid from an agropine strain. The T-DNA contains two segments, T_{L} and T_{R}, which are separated by a 15Kb sequence that is not integrated. The T_{L}-DNA contains 18 open reading frames (ORFs) where the four root loci-genes reside. The T_{R}-DNA contains several genes, including *aux1* and *aux2.*

"Hairy root phenotype" refers to a plant phenotype indicative of a putative transformed plant. That is, when *A. rhizogenes* infects a plant cell and transfer one or more *rol* genes, hairy root growth occurs at the infection site. In this way, a hairy root phenotype offers a marker-free method for identifying putative transformants.

"Intermediate height" refers to a quantitative reduction of plant height relative to the height of a wild-type or control plant of the same species but to an increase in plant height relative to the height of a plant of the said species transformed with one or more genes of the Ri plasmid of *Agrobacterium rhizogenes.* As transformed plants often display compact growth or dwarfism, the height of a transformed plant is usually about by less than 70%, 65%, 60%, 50%, 40, 30% or even less than 25%, 20%, 15% or 10% of the height of a wild type non transformed control plant. So in case a transformed plant displays a height of 30% as compared to the height of a wild type untransformed plant of the same species, preferably a parent plant from which the transformed plant originates, plants of 'intermediate height' are defined by a length of more than 30% of the height of the wild type untransformed plant, but having a height of less than the height of the said wild type untransformed plant.

The term "control plant" is intended to mean a same plant as a transformant plant, i.e. originating from the same parental source or belonging to the same species or variety. The skilled person will immediately understand how to compare transformed plant with control plants and what plants to take as control plant.

### A. NUCLEIC ACID SEQUENCES

The term "gene" refers to a nucleic acid (e.g., DNA or RNA) sequence that comprises coding sequences necessary for the production of RNA or a polypeptide. A polypeptide can be encoded by a full-length coding sequence or by any part thereof. The term "parts thereof' when used in reference to a gene refers to fragments of that gene, particularly a fragment encoding at least a portion of a protein. The fragments may range in size from a few nucleotides to the entire gene sequence minus one nucleotide. Thus, "a nucleic acid sequence comprising at least a part of a gene" may comprise fragments of the gene or the entire gene.

"Gene" also encompasses the coding regions of a structural gene and includes sequences located adjacent to the coding region on both the 5' and 3' ends for a distance of about 1 kb on either end such that the gene corresponds to the length of the full-length mRNA. The sequences which are located 5' of the coding region and which are present on the mRNA are referred to as 5' non-translated (or untranslated) sequences (5' UTR). The sequences which are located 3' or downstream of the coding region and which are present on the mRNA are referred to as 3' non-translated (or untranslated) sequences (3' UTR).

"Nucleic acid" as used herein refers to RNA or DNA that is linear or branched, single or double stranded, or a hybrid thereof. The term also encompasses RNA/DNA hybrids.

"Encoding" and "coding" refer to the process by which a gene, through the mechanisms of transcription and translation, provides information to a cell from which a series of amino acids can be assembled into a specific amino acid sequence to produce an active enzyme. Because of the degeneracy of the genetic code, certain base changes in DNA sequence do not change the amino acid sequence of a protein. It is therefore understood that modifications in the DNA sequence encoding transcription factors which do not substantially affect the functional properties of the protein are contemplated.

The term "expression," as used herein, refers to the production of a functional end-product e.g., an mRNA or a protein.

The terms "polypeptide," "peptide" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers.

Probe or primer refers to a short oligonucleotide sequence that could be designed and synthesized or generated as a fragment of a larger sequence. A probe or primer can be any length, such as 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 nucleotides in length.

Illustrative *rol* sequences include but are not limited to the sequences set forth in SEQ ID NOs: 1-18, respectively, as well as nucleic acid molecules comprised of fragments or variants of SEQ ID NO: 1-18 with one or more bases deleted, substituted, inserted, or added, which variant codes for a polypeptide with *rol* activity. For example, and in no way limiting, the present disclosure provides SEQ ID NO: 1, as well as various fragments of SEQ ID NO: 1, which could include, for example, *rol*A-D and *aux*1-2*.* For instance, and as readily apparent to one of ordinary skill in the art, the *rol*A gene could represent a 700 bp portion or fragment of a larger sequence comprising *rol*A-D and *aux*1-2*.*

A "variant" is a nucleotide or amino acid sequence that deviates from the standard, or given, nucleotide or amino acid sequence of a particular gene or protein. The terms "isoform," "isotype," and "analogue" also refer to "variant" forms of a nucleotide or an amino acid sequence. An amino acid sequence that is altered by the addition, removal, or substitution of one or more amino acids, or a change in nucleotide sequence, may be considered a "variant" sequence. The variant may have "conservative" changes, wherein a substituted amino acid has similar structural or chemical properties, e.g., replacement of leucine with isoleucine. A variant may have "nonconservative" changes, e.g., replacement of a glycine with a tryptophan. Analogous minor variations may also include amino acid deletions or insertions, or both. Guidance in determining which amino acid residues may be substituted, inserted, or deleted may be found using computer programs well known in the art such as Vector NTI Suite (InforMax, Md.) software. "Variant" may also refer to a "shuffled gene" such as those described in Maxygen-assigned patents.

Included in the category of "variant" sequences are sequences that hybridize to a reference *rol* sequence. For example, two sequences hybridize when they form a double-stranded complex in a hybridization solution of 6x SSC, 0.5% SDS, 5x Denhardt's solution and 100 µg of non-specific carrier DNA. See Ausubel et al., supra, at section 2.9, supplement 27 (1994). Sequences may hybridize at "moderate stringency," which is defined as a temperature of 60°C in a hybridization solution of 6 x SSC, 0.5% SDS, 5.times. Denhardt's solution and 100 µg of non-specific carrier DNA. For "high stringency" hybridization, the temperature is increased to 68°C. Following the moderate stringency hybridization reaction, the nucleotides are washed in a solution of 2 x SSC plus 0.05% SDS for five times at room temperature, with subsequent washes with 0.1 x SSC plus 0.1% SDS at 60°C for 1 hour. For high stringency, the wash temperature is increased to 68°C. One with ordinary skill in the art can readily select such conditions by varying the temperature during the hybridization reaction and washing process, the salt concentration during the hybridization reaction and washing process, and so forth. For present purposes, hybridized nucleotides can be detected using 1 ng of a radiolabelled probe having a specific radioactivity of 10,000 cpm/ng, where the hybridized nucleotides are clearly visible following exposure to X-ray film at -70°C for no more than 72 hours.

The present application is directed to such nucleic acid molecules that are at least 60%, 65%, 70%, 75%, 80%, 85%, 90%, 95%, 96%, 97%, 98%, 99% or 100% identical to a nucleic acid sequence described in any of SEQ ID NO: 1-18. Preferred are nucleic acid molecules which are at least 95%, 96%, 97%, 98%, 99% or 100% identical to the nucleic acid sequence shown in any of SEQ ID NO: 1-18. Differences between two nucleic acid sequences may occur at the 5' or 3' terminal positions of the reference nucleotide

As a practical matter, stating whether any particular nucleic acid molecule is at least 95%, 96%, 97%, 98% or 99% identical to a reference nucleotide sequence implicates a comparison made between two molecules, using algorithms known in the art and can be determined conventionally using publicly available computer programs such as the BLASTN algorithm. See Altschul et al., Nucleic Acids Res. 25: 3389-402 (1997).

The terms "sequence identity" and "sequence similarity" can be determined by alignment of two peptide or two nucleotide sequences using global or local alignment algorithms. Sequences may then be referred to as "substantially identical" or "essentially similar" when they share at least 70% of sequence identity over their entire length, respectively. Sequence alignments and scores for percentage sequence identity may be determined using computer programs, such as the GCG Wisconsin Package, Version 10.3, available from Accelrys Inc., 9685 Scranton Road, San Diego, Calif. 92121-3752 USA, or EmbossWin version 2.10.0 (using the program "needle"). Alternatively, percent similarity or identity may be determined by searching against databases, using algorithm as FASTA, BLAST, etc.

The present disclosure may contemplate nucleic acid molecules encoding functional proteins. As known in the art, it is understood that such proteins encompass amino acid substitutions, additions, and deletions that do not alter the function of any of the proteins.

Because many proteins are encoded by gene families, it is expected that other genes could encode proteins with similar functions as the instant polypeptides. These genes can be identified and functionally annotated by sequence comparison. A worker skilled in the art can identify a functionally related protein sequence with the aid of conventional methods such as screening cDNA libraries or genomic libraries with suitable hybridization probes. The skilled artisan knows that paralogous sequences can also be isolated with the aid of (degenerate) oligonucleotides and PCR-based methods.

### B. NUCLEIC ACID CONSTRUCTS

As explained above, one or more gene sequences originating from a Ri plasmid of *Agrobacterium rhizogenes* are transferred into a host plant cell. Such transfer can occur through natural means, such as natural infection of plant cell with *A. rhizogenes* carrying the Ri plasmid including native *rol* genes. Such natural or native transfer avoids the need for constructs and selection markers.

However, in another aspect, one or more one or more genes originating from a Ri plasmid of *Agrobacterium rhizogenes,* including *rol* sequences can be incorporated into a nucleic acid construct that is suitable for introduction into a plant cell. Thus, in instance where a native system is not employed, a nucleic acid construct can be used to express *rol* in a plant cell.

Exemplary nucleic acid constructs may comprise a base sequence of a minimum length to generate a mRNA and consequently a polypeptide. There is no theoretical upper limit to the base sequence length. The preparation of such constructs is described in more detail below.

As a source of the nucleic acid sequence for transcription, a suitable cDNA or genomic DNA or synthetic polynucleotide may be used. Methods for the isolation of suitable *rol* sequences are described, supra. Sequences coding for the whole, or substantially the whole, of the sequence may thus be obtained. Suitable lengths of this DNA sequence may be cut out for use by means of restriction enzymes. When using genomic DNA as the source of a partial base sequence for transcription, it is possible to use either intron or exon regions or a combination of both.

Recombinant nucleic acid constructs may be made using standard techniques. For example, the nucleic acid sequence for transcription may be obtained by treating a vector containing said sequence with restriction enzymes to cut out the appropriate segment. The nucleic acid sequence for transcription may also be generated by annealing and ligating synthetic oligonucleotides or by using synthetic oligonucleotides in a polymerase chain reaction (PCR) to give suitable restriction sites at each end. The nucleic acid sequence then is cloned into a vector containing suitable regulatory elements, such as upstream promoter and downstream terminator sequences.

Another aspect concerns a nucleic acid construct wherein a gene sequence of one or more genes originating from a Ri plasmid of *Agrobacterium rhizogenes,* such as a *rol* sequence is operably linked to one or more regulatory sequences, which drive expression of the *rol* sequence in certain cell types, organs, or tissues without unduly affecting normal development or plant physiology.

Of course, and in the context of a natural transformation or natural infection system, native or endogenous regulatory sequences are used, rather than heterologous sequences.

"Promoter" connotes a region of DNA upstream from the start of transcription that is involved in recognition and binding of RNA polymerase and other proteins to initiate transcription. A "constitutive promoter" is one that is active throughout the life of the plant and under most environmental conditions. Tissue-specific, tissue-preferred, cell type-specific, and inducible promoters constitute the class of "non-constitutive promoters." "Operably linked" refers to a functional linkage between a promoter and a second sequence, where the promoter sequence initiates and mediates transcription of the DNA sequence corresponding to the second sequence. In general, "operably linked" means that the nucleic acid sequences being linked are contiguous.

Promoters useful for expression of a nucleic acid sequence introduced into a cell may include native or endogenous promoters for natural transformation systems, or constitutive promoters, such as the cauliflower mosaic virus (CaMV) 35S promoter, or tissue-specific, tissue-preferred, cell type-specific, and inducible promoters. For example, by using vascular system-specific, xylem-specific, or xylem-preferred promoters, one can modify *rol* expression specifically in many tissues such as vascular tissues, especially xylem. The use of a constitutive promoter in general affects enzyme levels and functions in all parts of the plant, while use of a tissue-preferred promoter permits targeting of the modified gene expression to specific plant parts, leading to a more controllable phenotypes.

A vector may also contain a termination sequence, positioned downstream of a *rol* sequence, such that transcription of mRNA is terminated, and polyA sequences added. Exemplary of such terminators are native or endogenous terminator sequences, cauliflower mosaic virus (CaMV) 35S terminator, or the nopaline synthase gene (Tnos) terminator. The expression vector also may contain enhancers, start codons, splicing signal sequences, and targeting sequences.

Expression vectors may also contain a selection marker by which transformed cells can be identified in culture. The marker may be associated with the heterologous nucleic acid molecule, i.e., the gene operably linked to a promoter. As used herein, the term "marker" refers to a gene encoding a trait or a phenotype that permits the selection of, or the screening for, a plant or cell containing the marker. In plants, for example, the marker gene will encode antibiotic or herbicide resistance. This allows for selection of transformed cells from among cells that are not transformed or transfected.

Examples of suitable selectable markers include adenosine deaminase, dihydrofolate reductase, hygromycin-B-phosphotransferase, thymidine kinase, xanthine-guanine phospho-ribosyltransferase, glyphosate and glufosinate resistance, and aminoglycoside 3'-O-phosphotranserase (kanamycin, neomycin and G418 resistance). These markers may include resistance to G418, hygromycin, bleomycin, kanamycin, and gentamicin. The construct may also contain the selectable marker gene Bar that confers resistance to herbicidal phosphinothricin analogues like ammonium glufosinate. Thompson et al., EMBO J. 9: 2519-23 (1987). Other suitable selection markers are known as well.

Visible markers such as green florescent protein (GFP) may be used. Methods for identifying or selecting transformed plants based on the control of cell division have also been described. See WO 2000/052168 and WO 2001/059086. Likewise, the presence of a distinguishing phenotype, such as tumour or hairy root growth, may also be used for identification and selection.

In a natural transformation or natural infection system, a selection marker is not employed. Because infection provides its own distinct and natural phenotype, a transformed cell can be selected based on a post-infection phenotype, such as hairy root phenotype.

Replication sequences, of bacterial or viral origin, may also be included to allow the vector to be cloned in a bacterial or phage host. Preferably, a broad host range prokaryotic origin of replication is used. A selectable marker for bacteria may be included to allow selection of bacterial cells bearing the desired construct. Suitable prokaryotic selectable markers also include resistance to antibiotics such as kanamycin or tetracycline.

Other nucleic acid sequences encoding additional functions may also be present in the vector, as is known in the art. For instance, when *Agrobacterium* is the host, T-DNA sequences may be included to facilitate the subsequent transfer to and incorporation into plant chromosomes.

### C. TRANSFORMATION METHODOLOGY: TRANSFER OF GENES

As explained above, transformation" refers to any methodology for introducing one or more genes originating from *Agrobacterium rhizogenes* into a host plant or plant cell. Importantly, and because *A. rhizogenes* naturally infects plants, transformation embraces transferring wild-type genes from the Ri plasmid from wild-type bacterium into a plant cell, in particular one or more *rol* genes and/or *aux* genes. Thus, and as used herein, transformation does not require cloning a heterologous gene into a vector for transfer into a host plant cell, nor does transformation require genetically engineering the bacterium.

"Transformed plant" refers to a plant that comprises a nucleic acid sequence that also is present per se in another organism or species, or that is optimized, relative to host codon usage, from another organism or species. Both monocotyledonous and dicotyledonous angiosperm or gymnosperm plant cells may be transformed in various ways known to the art. For example, see Klein et al., Biotechnology 4: 583-590 (1993); Bechtold et al., C. R. Acad. Sci. Paris 316: 1194-1199 (1993); Bent et al., Mol. Gen. Genet. 204: 383-396 (1986); Paszowski et al., EMBO J. 3: 2717-2722 (1984); Sagi et al., Plant Cell Rep. 13: 262-266 (1994). *Agrobacterium* species such as *A. tumefaciens* and *A. rhizogenes* can be used, for example, in accordance with Nagel et al., Microbiol Lett 67: 325 (1990). Additionally, plants may be transformed by *Rhizobium, Sinorhizobium* or *Mesorhizobium* transformation. Broothaerts et al., Nature 433: 629-633 (2005).

For example, *Agrobacterium* may be transformed with a plant expression vector via, e.g., electroporation, after which the *Agrobacterium* is introduced to plant cells via, e.g., the well-known leaf-disc method. Additional methods for accomplishing this include, but are not limited to, electroporation, particle gun bombardment, calcium phosphate precipitation, and polyethylene glycol fusion, transfer into germinating pollen grains, direct transformation, Lorz et al., Mol. Genet. 199: 179-182 (1985), and other methods known to the art. If a selection marker, such as kanamycin resistance, is employed, it makes it easier to determine which cells have been successfully transformed. Marker genes may be included within pairs of recombination sites recognized by specific recombinases such as cre or flp to facilitate removal of the marker after selection. See U.S. published application No. 2004/0143874.

Transgenic plants without marker genes may be produced using a second plasmid comprising a nucleic acid encoding the marker, distinct from a first plasmid that comprises a gene sequence originating from the *Agrobacterium rhizogenes* Ri plasmid, in particular a *rol* sequence. The first and second plasmids or portions thereof are introduced into the same plant cell, such that the selectable marker gene that is transiently expressed, transformed plant cells are identified, and transformed plants are obtained in which the envisaged gene sequence, such as a *rol* sequence is stably integrated into the genome and the selectable marker gene is not stably integrated. See U.S. published application No. 2003/0221213.

The *Agrobacterium* transformation methods discussed above are known for transforming dicots. Additionally, de la Pena et al., Nature 325: 274-276 (1987), Rhodes et al., Science 240: 204-207 (1988), and Shimamato et al., Nature 328: 274-276 (1989) have transformed cereal monocots using *Agrobacterium.* Also see Bechtold et al., C.R. Acad. Sci. Paris 316 (1994), illustrating vacuum infiltration for Agrobacterium-mediated transformation.

Plant cells may be transformed with a nucleic acid or nucleic acid construct without the use of a selectable or visible marker, and transgenic organisms may be identified by detecting the presence of the introduced sequence or construct. The presence of a protein, polypeptide, or nucleic acid molecule in a particular cell can be measured to determine if, for example, a cell has been successfully transformed or transfected. For example, and as routine in the art, the presence of the introduced construct can be detected by PCR or other suitable methods for detecting a specific nucleic acid or polypeptide sequence. Additionally, transformed cells may be identified by recognizing differences in the growth rate or a morphological feature of a transformed cell compared to the growth rate or a morphological feature of a non-transformed cell that is cultured under similar conditions. See WO 2004/076625.

Methods of regenerating a plant from a transformed cell or culture vary according to the plant species but are based on known methodology. For example, methods for regenerating *Kalanchoe* plants are well-known in the art can be found in Christensen, et al, (2008). Plant Cell Rep. 27, 1485-1495.

### D. SELECTION AND ANALYSIS OF PLANTS TRANSFORMED WITH A.RHIZOGENES

The present transformed plants are selected that contain and express one or more genes originating from the Ri plasmid of *Agrobacterium rhizogenes* relative to a control, non-transformed plant of the same species, said genes preferably comprising one or more *rol* genes. Additionally, the instant plants may have an altered phenotype relative to a non-transformed control plant. Such phenotype may include an intermediate height or intermediate compactness, wherein the transformed plant has a reduced height and/or compactness relative to the control plant.

The present inventors have now, in a first aspect of the invention, been capable to provide plants of intermediate height, without the need of being subjected to the drawbacks of using chemical growth retarding agents, and while still capable of flowering as comparable to the wild type control plants, with a similar number of flowers or even an increased number of flowers as compared to the flower number of control plants. To this end, the invention provides a method for obtaining a plant having a height of 50 - 75% of the height of a corresponding wild type plant, and not less than 80% of the number of flowers thereof, comprising the steps of:
(a) transforming tissue of the wild type plant with one or more genes originating from the Ri plasmid of *Agrobacterium rhizogenes,*
(b) allowing the transformed tissue to develop roots,
(c) selecting a putatively transformed root having a hairy root phenotype;
(d) growing the selected root on a regeneration medium;
(e) allowing a transformed rooted plantlet to generate from said root;
(f) growing said transformed rooted plantlet into a mature transformed mother plant having a height of less than 40% of that of the corresponding wild type plant,
(g) preparing a transformed cutting from the mature transformed mother plant,
(h) allowing a rooted transformed plantlet to develop from the transformed cutting,
(i) treating the rooted transformed plantlet with gibberellin, therewith allowing the plantlet to develop in a mature plant having a height between the height of the transformed mother plant of step (f) and the height of the wild type plant of step (a).

It has surprisingly found that plants transformed with one or more genes originating from the Ri plasmid of *Agrobacterium rhizogenes,* and treated with gibberellin during the development to a mature plant display the envisaged intermediate growth as defined above, and may as well not have the above disadvantages or to a significant lesser extent. Herein, the phrase 'originating from *Agrobacterium rhizogenes'* is particularly intended to mean 'originating from the Ri plasmid of *Agrobacterium rhizogenes',* in particular from the T_{L}- and T_{R}-DNA of the Ri plasmid as shown in SEQ ID NOs 1 and 2, respectively.

In step (a), tissue of the wild type plant is transformed with one or more genes originating from the Ri plasmid of *Agrobacterium rhizogenes.* As indicated above, the skilled person is aware about how to introduce said genes in the plant in such a way that the said gene or genes are expressed. The plant can e.g. be obtained by infection with *Agrobacterium rhizogenes,* e.g. by contacting a culture of *Agrobacterium rhizogenes* with a fresh wound of the plant or a portion thereof. However, it is also possible to introduce into plant cell one or more *Agrobacterium rhizogenes* genes of the Ri plasmid, or a combination of two or more thereof, such as one or more *rol* genes, a combination of two or more thereof, or all four *rol* genes, into the plant cells by known genetic engineering techniques. Infection with *Agrobacterium rhizogenes* of a plant or plant cells, however, has been proven very efficient and convenient. The skilled person is aware of proper techniques as how to transfect an envisaged plant with *Agrobacterium rhizogenes,* and how the produce transformed plants , see e.g. Christensen et al., *supra.*

In a subsequent step, the thus transformed tissue is allowed to develop roots, followed by selecting roots with hairy root phenotype, a well-known phenotypic selection marker for *A. rhizogenes* transformed plant tissue. By growing the selected root on a regeneration medium (e.g. Christensen et al., supra), transformed rooted plantlets are allowed to be generated. One or more shoots are formed from the root, resulting in transformed rooted plantlets.

The said rooted plantlet is grown into a mature mother plant having a height of less than 40% of that of the corresponding wild type plant. The term 'mature' intends to mean that the plant will be capable to be brought to sexual reproduction, in contrast to 'juvenile', herein meaning that the plant has not yet developed to a stage wherein it is capable to be brought to sexual reproduction. This means that the plant must be in a development stage that flowers can be generated. As indicated earlier, transformed plants will display a compact or dwarfism growth behaviour, resulting in a plant height that is significantly less than that of the originating non transformed wild type plant. It is to be understood that in case transformed mother plants are found having a height of 40% or more as compared to the height of the originating non transformed wild type plant, such plant is not selected for preparing cuttings from in the subsequent step. Therefore, step (f) may comprise the step of selecting a mature mother plant having a height of less than 40% of that of the corresponding wild type plant.

Subsequently, transformed cuttings are prepared from the mature transformed plant. It is also possible to take cuttings from progeny of the said mature transformed mother plant, such as from a mature plants, originating from a cutting form the said mature mother plant. It is also possible to cross the mature transformed mother plant with another, possible not transformed plant of the same species, or from another species, i.e. generating a transformed mature interspecific hybrid mother plant.

The cuttings are kept and treated such that rooted transformed plantlets develop from the cuttings. Said plantlets are young plants in the process of becoming a mature plant. During this stage of developing in a mature plant, the rooted transformed plantlet is treated with gibberellin, a plant growth hormone, therewith allowing the plantlet to elongate and develop in a mature plant having a height between the height of the transformed mother plant of step and the height of the initial wild type plant. Without the gibberellin treatment, the plantlet would develop in a plant, similar or identical in height to the transformed mother plant. The gibberellin treatment surprisingly results in plants of intermediate height, capable of forming a similar or even a higher number of flowers as compared to wild type control plants, with the capability to flower even earlier and longer both compared to the originating wild type plant as well as compared to the transformed mother plant.

It was also found that by gibberellin treatment, the internode distance increased as compared to the mature mother plant, facilitating the preparation of cuttings. *A. rhizogenes* transformed mother plants displaying a dwarfism type of growth behaviour were difficult to obtain cuttings from without damaging the mother plants.

In an attractive embodiment, step (a) comprises co-cultivating *A. rhizogenes* with a plant or plant part, such as a leaf or leaf portion, allowing *A. rhizogenes* to deliver the said one or more genes of the Ri plasmid into said plant or plant portion. As indicated above, the one or more genes, such as one or more rol genes can be introduced in the plant cells by any known transformation technique, it is preferred to co-cultivate *A. rhizogenes* with a plant or plant portion, e.g. including wounding the plant or plant part.

Preferably, the one or more genes originating from the Ri plasmid of *Agrobacterium rhizogenes* comprises one or more *rol* genes, in particular chosen from *rol*A*, rol*B and *rol*C as described above. However, also *rol*D*, aux*1*, aux*2 and any of the open reading frames as identified on the Ri plasmid can be included. Based on the above-mentioned literature and experimental data is believed that in particular *rol* genes may play an important role in the above-described characteristics. Therefore, it is preferred to transform the plant in step (a) with at least *rol*A*, rol*B and *rol*C genes, preferably also with *rol*D*.*

In a preferred embodiment, step (a) comprises infection with *Agrobacterium rhizogenes,* which is a natural way of transformation without the need of applying genetic manipulation techniques. While any methodology can be used for transforming plants with *A. rhizogenes,* in particular with one or more *rol* genes, the present disclosure provides both "natural" and "non-natural" methodology for generating transformed plants, in particular belonging to any of the genera as described below. For example, and as discussed below, applicants harnessed wild-type *A. rhizogenes* to transfer its native genes, including its *rol* genes into a plant cell. While this is a "natural" system in that A. *rhizogenes* transfers its native genes to plant cells, it is unlikely to successfully occur in nature because compact plants transformed with *Agrobacterium rhizogenes* face obstacles such as increased risk of fungal infection due to compact leaves forming closer structures, as well as competitiveness from neighbouring plants.

The *Agrobacterium rhizogenes* is preferably wild-type, i.e. not containing genetic material that has been introduced by way of genetic engineering. Advantageously, using wild-type *Agrobacterium rhizogenes* results in transformants that are to be regarded as not being a GMO (genetically modified organism).

In a preferred embodiment, the mature transformed mother plant of step (f) has a height of less than 65%, preferably less than 50%, and more preferably less than 25% of the height of the wild type plant of step (a). Such heights are obtained when plants, transformed by *A. rhizogenes* are allowed to grow to mature plants. The advantage of the invention become more pronounced when transformation of wild type plants results in dwarfism growth behaviour, as it is difficult to produce cuttings from such dwarf plants, and the need for plants of intermediate height is higher for dwarf plants.

In a very attractive embodiment, step (f) further comprises treating the transformed rooted plantlet with gibberellin before the transformed rooted plantlet reaches maturity. This results in a mature transformed mother plant that is itself of intermediate height, so that taking cuttings in the subsequent step is facilitated. Although the gibberellin treatment can be performed only once, it may be preferred to repeat the gibberellin treatment before the plantlet reaches maturity, in particular in case the transformed plant is very small, and the difference between the height of the transformed mother plant and the envisaged intermediate plant height is significant. The skilled person is aware of suitable gibberellin treatments. For gibberellin treatments, e.g. the product Berelex (Valent BioSciences, Libertyville, IL, USA) can be used, as will be discussed below. One or more treatments can be applied to the plants in their growing cycle, depending on the height of the plant as compared to the non-transformed control plant and the envisaged intermediate height to be obtained.

In a preferred embodiment, step (i) comprises allowing the plantlet to develop in a mature plant having a height of up to 75%, preferably of up to 70%, more preferably of up to up to 65% of the height of the wild type plant of step (a). So, if a wild type plant, e.g. a *Kalanchoe* cultivar has a height of e.g. 40 cm, the method preferably provides a plant having an intermediate height of up to 30 cm, preferably up to 28 cm, more preferably of up to 26 cm.

On the other hand, step (i) preferably comprises allowing the plantlet to develop in a mature plant having a height of at least 40%, preferably at least 50% of the height of the wild type plant of step (a). In the above example of a wild type pant of 40 cm, the envisaged plant obtained by the described method preferably has an intermediate height of at least 16 cm, more preferably of at least 20 cm.

The wild type plant of step (a) preferably belongs to any of the following genera or species:
*Kalanchoe,* in particular *K*. *blossfeldiana, K. laciniata, K. pinnata, K. marmorata, K. gastonis-bonnieri, K. dixoniana, K. humilis, K*. *ambolensis, K*. *aromatica, K. campanulata, K. citrina, K. coccinea, K. crundallii, K. daigremontiana, K. decumbens, K. faustii, K. fedtschenkoi, K. figueredoi, K. flammea, K*. *glaucescens, K. gracilipes, K. grandiflora, K. guignardii, K. jongmansii, K. laciniata, K. latisepela, K. laxiflora, K. lobata, K. longiflora, K. manginii, K. nyikae, K. obtuse, K. paniculata, K. porphyrocalyx, K. prittwitzii, K. pubescens, K. pumila, K. rauhii, K. rotundifolia, K. scapigera, K. schumacherii, K. spathulata, K. streptantha, K. synsepala, K*. *tomentosa, K*. *thyrsiflora, K. tubiflora, K. uniflora;*
*Chrysantemum,* in particular *Chrysantemum morifolium, Chrysantemum x morifolium (syn. C. x grandiflorum e.g.Dendranthema hybrids, or hybrids between Chrysantemum morifolium* and other *Chrysantemum* species, such as *Chrysanthemum indicum;*
*Aster,* in particular *Aster novi-belgii, Aster dumosus;*
*Rosa,* in particular *Rosa hybrida, Rosa canina, Rosa spinosissima, Rosa damascena "trigintipetala', Rosa centifolia;*
*Solanum,* in particular *Solanum lycopersicum, Solanum tuberosum, Solanum nicotiana;*
*Euphorbia,* in particular *Euphorbia pulcherrima, Euphorbia milii*;
*Phalaenopsis,* in particular *Phalaenopsis amabilis, Phalaenopsis amboinensis, Phalaenopsis aphrodite, Phalaenopsis appendiculata;*
*Ocimum,* in particular *Ocimum basilicum;*
*Capsicum,* in particular *Capsicum annuum, Capsicum baccatum, Capsicum chinense, Capsicum frutescens, Capsicum pubescens;*
*Mentha,* in particular *Mentha arvensis, Mentha requienii, Mentha spicata, Mentha longifolia, Mentha pulegium, Mentha suaveolens, Mentha aquatic, Mentha arvensis x spicata, Mentha aquatica x arvensis, Mentha x piperita;*
*Hibiscus,* in particular *Hibiscus rosa-sinensis, Hibiscus schizopetalus, Hibiscus sabdariffa, Hibiscus syriacus, Hibiscus trionum, Hibiscus cannabinus;*
*Mandevilla*/*Dipladenia,* in particular *Mandevilla xamabilis, Mandevilla sanderi, Mandevilla splendens;*
*Eustoma,* in particular *Eustoma russellianum, Eustoma exaltatum;*
*Lavendula,* in *particular Lavandula angustifolia, Lavandula latifolia, Lavandula lanata,Lavandula dentate, Lavandula stoechas, Lavandula pedunculata, Lavandula viridis*;
*Lillium,* in particular *Lilium bolanderi, Lilium* × *burbankii, Lilium canadense, Lilium columbianum, Lilium grayi, Lilium humboldtii, Lilium kelleyanum, Lilium kelloggii, Lilium maritimum, Lilium michauxii, Lilium michiganense, Lilium occidentale, Lilium* × *pardaboldtii, Lilium pardalinum, Lilium parryi, Lilium parvum, Lilium philadelphicum, Lilium pitkinense, Lilium superbum, Lilium ollmeri, Lilium washingtonianum, Lilium wigginsii*;
*Clematis,* in particular *Clematis addisonii, Clematis albicoma, Clematis alpine, Clematis aristata, Clematis armandii, Clematis baldwinii,Clematis bigelovii, Clematis brachiate, Clematis campaniflora, Clematis catesbyana, Clematis chinensis, Clematis chrysocoma, Clematis cirrhosa, Clematis coactilis, Clematis Columbiana, Clematis crispa, Clematis dioica, Clematis drummondii, Clematis durandii, Clematis ispahanica, Clematis fawcettii, Clematis flammula, Clematis florida, Clematis fremontii, Clematis glaucophylla, Clematis glycinoides, Clematis henryi Clematis hirsutissima, Clematis integrifolia, Clematis* × *jackmanii, Clematis lanuginose, Clematis lasiantha, Clematis leptophylla, Clematis ligusticifolia, Clematis macropetala, Clematis marmoraria, Clematis microphylla, Clematis montana, Clematis morefieldii, Clematis napaulensis, Clematis occidentalis, Clematis ochroleuca, Clematis orientalis, Clematis palmeri, Clematis, Clematis patens, Clematis pauciflora, Clematis pickeringii, Clematis pitcher, Clematis recta, Clematis reticulate, Clematis rhodocarpa, Clematis smilacifolia, Clematis socialis, Clematis stans, Clematis tangutica, Clematis terniflora, Clematis ternifolia, Clematis texensis, Clematis versicolor, Clematis viorna, Clematis virginiana, Clematis vitalba, Clematis viticaulis, Clematis viticella;*
*Geranium,* in particular *Geranium cinereum, Geranium clarkei, Geranium dalmaticum, Geranium endressii, Geranium fremontii, Geranium himalayense, Geranium ibericum, Geranium macrorrhizum, Geranium maculatum, Geranium maderense, Geranium* × *magnificum, Geranium phaeum, Geranium platypetalum, Geranium pretense, Geranium psilostemon, Geranium renardii, Geranium sanguineum, Geranium subcaulescens, Geranium sylvaticum;*
*Pelargonium,* in particular *Pelargonium crispum, Pelargonium grandiflorum, Pelargonium peltatum, Pelargonium graveolens, Pelargonium denticulatum, Pelargonium x hortorum,, Pelargonium x asperum;*
*Nicotiana,* in particular *Nicotiana tabacum, Nicotiana sylvestris, Nicotiana x sanderrae;*
*Bouvardia,* in particular *Bouvardia longiflora;*
*Vanilla,* in particular *Vanilla planifolia;*
*Ipomoea,* in particular *Ipomoea batatas;*
*Echinacea,* in *particular Echinacea purpurea, Echinacea angustifolia, Echinacea pallida;*
*Schisandra,* in particular *Schisandra chinensis, Schisandra glabra, Schisandra rubriflora;*
*Rhodiola,* in particular *Rhodiola rosea;*
*Leucanthemum,* in particular *Leucanthemum maximum, Leucanthemum paludosum, Leucanthemum x superbum, Leucanthemum vulgare, Leucanthemum adustum, Leucanthemum graminifolium, Leucanthemum integrifolium, Leucanthemum lacustre, Leucanthemum monspeliense, Leucanthemum pallens, Leucanthemum praecox, Leucanthemum subglaucum, Leucanthemum sylvaticum, Leucanthemum waldsteinii;*
*Strelitzia,* in particular *Strelitzia reginae,* including interspecific hybrids within the genera, and progeny thereof. Intergeneric grafts of any of the above genera and interspecific grafts of any of the species within the genera are included as well.

In a particular embodiment, step (f) further comprises selecting a mature transformed mother plant, having not less than 80% of the branching as compared to the wild type plant of step (a) from which a transformed cutting is prepared in step (g). Although gibberellin treated transformed plants as described herein tend to have a similar number of flowers as compared to the originating wild type plant, this selection step enables the generation of plants of intermediate height with a significant number of flowers in a more controlled manner. Said branching is more preferably not less than 90%, most preferably not less than 100%, i.e. having equal or more flowers as compared to the wild type plant of step (a).

Preferably, the above method comprises a step of assaying the presence of one or more *rol* genes originating from the Ri plasmid of *Agrobacterium rhizogenes* in said mature transformed mother plant. A possible assay is explained above, but the skilled person will be aware of any suitable and applicable assays.

In view of attractive appearance, handling, storage and presentation, the mature plants of step (i) of the method preferably have a height of 10 - 30 cm, more preferably of 15 - 20 cm.

In an attractive embodiment, the above method further comprises the steps of subjecting, as a parent, the mature transformed plant of step (i) or progeny thereof, to a crossing event, and selection of the progeny. By further crossing the transformants, additional functions can be introduced, or present functions can be further improved or removed from the plant in later generations. The hybrids of the progeny can be further crossed and selected, and this can be repeated several round in order to arrive at the envisaged result.

In another embodiment, steps (a) - (f) are replaced by providing a mother plant, transformed with one or more genes originating from the Ri plasmid of *Agrobacterium rhizogenes,* having a height of 40% or less of that of the corresponding wild type plant. Instead of performing the transformation of step (a) and preparing a transformed mother plant in the subsequent steps (b) - (f), it is also possible to obtain the transformed mother plant from elsewhere. It can very well be possible to purchase e.g. a plant, transformed with *Agrobacterium rhizogenes,* having compact growth or dwarfism and use the said plant as transformed mother plant for performing steps (g) - (i) to provide mature plants having intermediate height.

In a second aspect, the invention relates to a plant, obtained by the described method, or progeny thereof, the plant or progeny comprising one or more genes originating from the Ri plasmid of *Agrobacterium rhizogenes.* The said plant preferably has a height of 25 - 75%, more preferably of 40 - 70% and most preferably of 50 - 65% of the height of the wild type plant of step (a) of the method. The height is preferably 10 - 30 cm, more preferably 15 - 20 cm.

The plant has preferably not less than 80% of the number of flowers and/or not having a delayed flowering time by more than 4 days as compared to a not transformed control plant. It has now found by the inventor that plants, transformed with *Agrobacterium rhizogenes,* in particular *rol* transformed plants of the above genera and species, including interspecific hybrids and intergeneric grafts thereof, surprisingly have no significant loss in flower number, i.e. having at least 80%, but preferably at least 90% or 100% or even more (i.e. an increased flower number) of the number of flowers as compared to a not transformed control plant. The same is true for the delay in flowering, being preferably not later than 4 days, more preferably not later than 2 days as compared to a not transformed plant. In some transformants, flowering occurred even earlier as compared to the not transformed control plants.

The plant can be for use as ornamental plant or as cut flower, in particular belonging to any of the genera *Strelitza, Aster, Chrysanthemum, Euphorbia, Hibiscus, Mandevilla, Nicotiana, Phalaenopsis, Rosa* and *Kalanchoe.*

The plant, can also be for use for the provision or preparation of food, or as source for an active ingredient for the preparation of a human or animal necessity, such as a medicament, a stimulant or a cosmetic. The term 'human or animal necessity' is meant herein to mean any product, comprising any plant part, compound, mixture of compounds or extract, derived from the said plant, for use by humans or animals, in particular by treatment of said humans or animals with such a product, in particular treatment of the body thereof, such as medical, cosmetical or stimulative treatment, or consumption of the product by the said animals or humans. Food as such is therefore also to be regarded as a human or animal necessity.

In the art, only visual evaluation of plants, transformed with *A. rhizogenes* including the field crop species *Cichorum intybus* (Belgian endive) and *Nicotiana tabacum* (tobacco) as source for an active ingredient for a stimulant (cigarettes) are described (Christensen and Müller, *supra*)*.* The present inventor has however surprisingly found that such plants, comprising one or more *Agrobacterium rhizogenes* genes of the Ri plasmid, in particular *rol* genes originating from *Agrobacterium rhizogenes,* such as plants transformed with *Agrobacterium rhizogenes* or one or more *rol* genes thereof, which have been treated with gibberellin as described herein, or progeny of such plants, not only have intermediate height, which is particularly desired when such plants grow very high, impairing harvesting, but also have more concentrated contents as a result of a smaller cell volume. Without being bound to any theory, is it believed that the smaller size of such gibberellin treated transformants is at least partially due to less elongating plant cells and thus smaller cells. This is pronounced in that e.g. the fruits of *rol* transformed fruits such as peppers from *Capsicum* species, or vanilla from *Vanilla planifolia,* or herbs, such as basil (*Ocimum basilicum*) have an intensified taste as compared to non-transformed counterparts. The same is true for tomato, potato, and monocots such as wheat, barley etc. The same is true for plants used as source for an active ingredient for the preparation of a medicament or a stimulant. Such plants, such as medicinal plants, have the active ingredient in a more concentrated fashion in their cells, therewith facilitating isolation and extraction thereof. In case of tobacco, comprising the stimulant nicotine, has a higher nicotine content, resulting in a more concentrated product. More details of use of plants, plant parts, compounds, and mixtures of compounds or extracts for use as human necessities are given in the examples.

The plant for use for the provision or preparation of food preferably belongs to any of the genera *Vanilla, Ocimum, Capsicum, Ipomoea, Solanum,* although any such plant is encompassed by the invention. The plant itself can be used as food, such as carrots, or fruits thereof, such as apples, pears, berries, etc. also, extracts can be prepared, such as e.g. tea.

The plant for use as source for an active ingredient for the preparation of a medicament or stimulant preferably belongs to any of the genera *Kalanchoe, Solanum, Nicotiana, Rhodiola, Echinacea, Schisandra, Rosa, Hibscus* and *Chrysanthemum. rol* transformed *Kalanchoe* comprises elevated levels of bryophillins, such as bryophillin A, having a strong anti-tumour promoting activity, whereas *rol* transformed *Schisandra* contains elevated levels of schisandrin, desoxyschisandrin, gomisins and pregomisin. *Echinacea, rol* transformed and treated with gibberellin, has elevated levels of e.g. propanoid and echinacoside.

Accordingly, the invention also relates to the use of a plant as described above, or part, extract or isolate thereof for the provision or preparation of food, or a human or animal necessity, such as a medicament, a stimulant or a cosmetic. For example, extract of leaves of *rol* transformed *Chrystantenum morfolium* or *Hibiscus sabdariffa* can be used for the preparation of tea, Rosa spp can be used for the preparation of perfumes etc.

In a very attractive aspect, the invention relates to a product, comprising a plant part, extract, compound, or a mixture of two or more thereof, derived from a plant as described herein or progeny thereof. Said product can e.g., be chosen from the group, consisting of fruits, tubers, roots, leaves, food extracts, cosmetics, medicaments, perfumes. But such product is not limited thereto. For example, fruits such as peppers or tomatoes, or tubers such as potatoes have a more intense taste as from plants, not being transformed with *Agrobacterium rhizogenes* and subsequently treated with gibberellin.

The following figures and examples are illustrative and do not limit the present application. Of course, it is understood that many variations and modifications can be made while remaining within the intended scope.

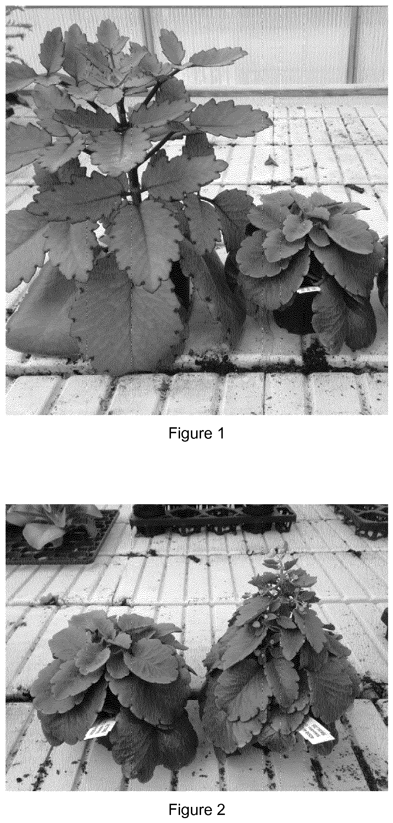

Figure 1 shows a wild type (left) and *Kalanchoe pinnata,* transformed with *Agrobacterium rhizogenes* (right), seven weeks after vegetative propagation long day conditions (light from 02:00 to 17:00). Three weeks after sticking the *Kalachoe pinnata* cutting the plants were moved to short day conditions for flower induction (light from 07:00 to 17:00). After 5 weeks under short day conditions, no flower buds were visible.
Figure 2 shows *Kalanchoe pinnata* ten weeks after vegetative propagation and 7 weeks into short day conditions for flower induction (light from 07:00 to 17:00) without (left) and with (right) gibberellin treatment. After 5 weeks and again after 7 weeks under short day conditions the plant to the right was sprayed to drop point with 1,25 mg GA3/L (ppm). The difference in flower formation between the untreated transformed *Kalanchoe pinnata* and the transformed *Kalanchoe pinnata* plant treated with GA3 is very clear. Flower formation and thus production time is significantly faster when rol transformed plants are treated with GA3.

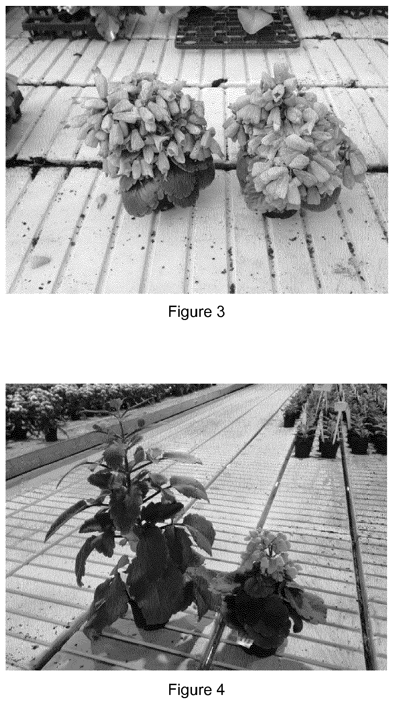

Figure 3 shows *Kalanchoe pinnata* of figure 2, 22 weeks after vegetative propagation and 19 weeks into short day conditions for flower induction (light from 07:00 to 17:00). After 5 weeks and again after 7 weeks under short day conditions the plant to the right was sprayed to drop point with 1,25 mg GA3/L (ppm). Flower formation did occur in both plants eventually, but much faster in the GA3 treated plant. Flower formation and thus production time is significantly faster when *rol* transformed plants are treated with GA3.
Figure 4 shows wild type (left) and *Kalanchoe pinnata,* transformed with *Argobacterium rhizogenes* (right), fifteen weeks after start of vegetative propagation and 12 weeks into short day conditions for flower induction (light from 07:00 to 17:00). After 5 weeks and again after 7 weeks under short day conditions the plants were sprayed to drop point with 1,25 mg GA3/L (ppm). The difference in flower formation between the wild type *Kalanchoe pinnata* (to the left) and the *rol* gene comprising transformed *Kalanchoe pinnata* plant (to the right) is very clear. Flower formation in the transformed *Kalanchoe pinnata* plant is very successful, whereas no flower formation is occurring in the wild type *Kalanchoe pinnata* (to the left).

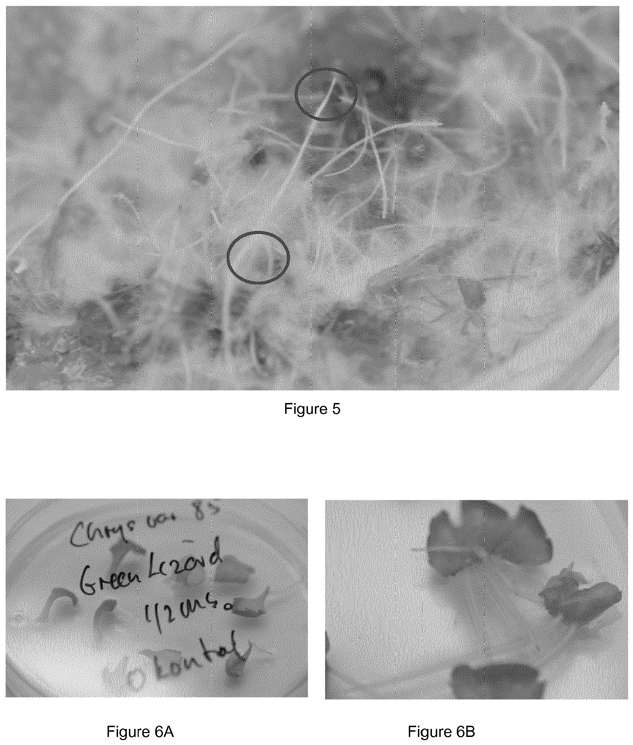

Figure 5 shows transformed *Solanum tuberosum* 'Bintje' roots. Notice highly branched roots with elongated root hairs. Plantlets have been regenerated from the transformed roots, marked with red circles.
Figure 6 shows *Chrysanthemum* 'var 85'. Figure 6A shows control leaflets that were not exposed to *Agrobacterium rhizogenes.* In the control plants only sporadic root formation, exclusively from petioles is observed. Figure 6B shows leaflets from where rol transformed hairy roots are formed (early stage). Root formation happens exclusively from the leaf perimeter.

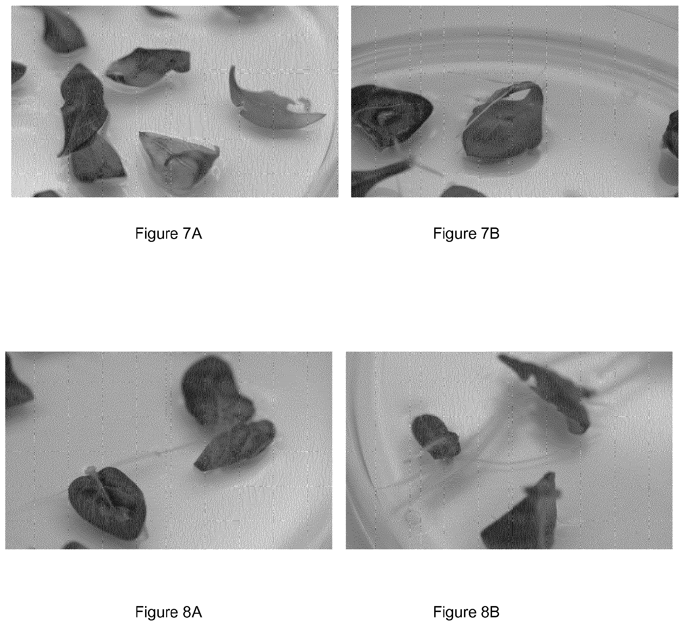

Figure 7 shows *Aster novi-belgii* '134A'. Figure 7A shows leaflets that have not been exposed to *Agrobacterium rhizogenes* and no root formation can be observed. Figure 7B shows early stages of root formation from the leaf perimeter.
Figure 8 shows *Solanum tuberosum* 'Bintje'. Figure 8A shows leaflets that have not been exposed to *Agrobacterium rhizogenes* and some root formation from petioles is observed. Figure 8B clearly shows that hairy root formation takes place (early stage) from the root perimeter, but also from the central surface of the leaflet.

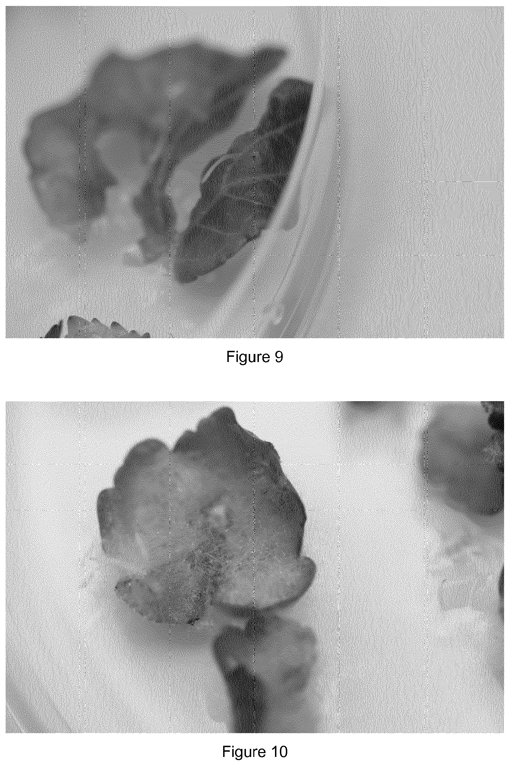

Figure 9 shows rol transformed *Hibiscus sabdariffe* roots. Root formation is occurring from the central surface of the leaflet.
Figure 10 shows transformed *Kalanchoe* interspecific hybrid 'NaomiQ2' roots. Transformed roots are extremely hairy and root branching is occurring at an early stage.

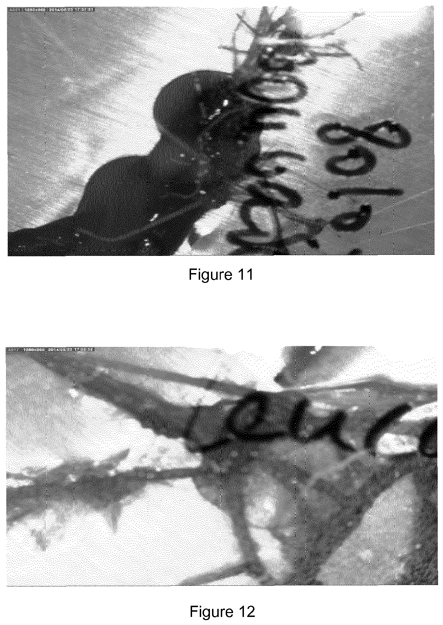

Figure 11 shows transformed *Bouvardia* hybrid '8010-0017' roots. Transformed root are extremely hairy and root branching is occurring at an early stage.
Figure 12 shows transformed *Leucanthemum* roots. The transformed roots are extremely hairy and root branching is occurring at an early stage.

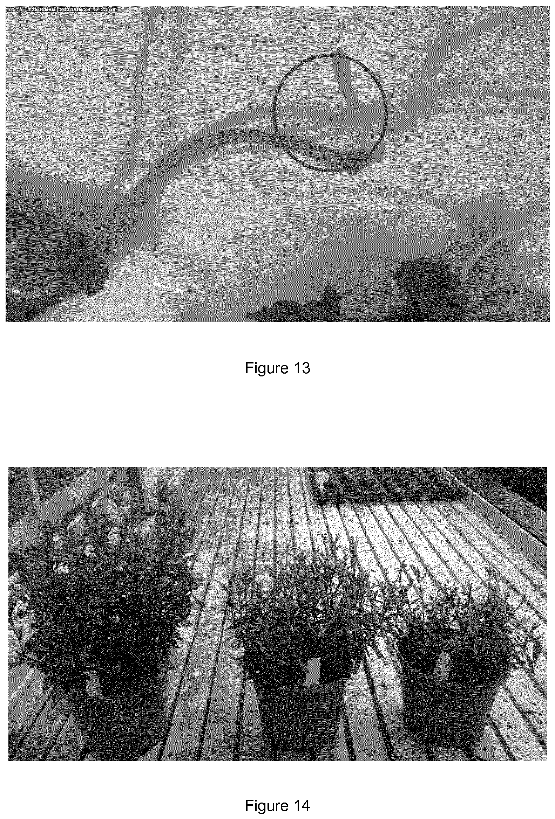

Figure 13 shows a shoot (red circle) regenerated from rol transformed root of Aster novi-belgii '134A'.
Figure 14 shows *Aster novi-belgii* '134A' mother plants wild type (left) and transformed with *Argobacterium rhizogenes* (centre and right), fifteen weeks after start of vegetative propagation. After 5 weeks and again after 7, 9 and 11 weeks the centre plant were sprayed to drop point with 0,1 mg GA3/L (ppm). The difference in growth between the wild type *Aster novi-belgii* '134A' (to the left) and the *rol* transformed *Aster novi-belgii* '134A' mother plant (to the right) is very clear. The *rol* transformed plants are significantly more compact. Formation and growth of side shoots are clearly more pronounced in the rol transformed plant treated with GA3 (centre) compared to the rol transformed control plant (to the right).

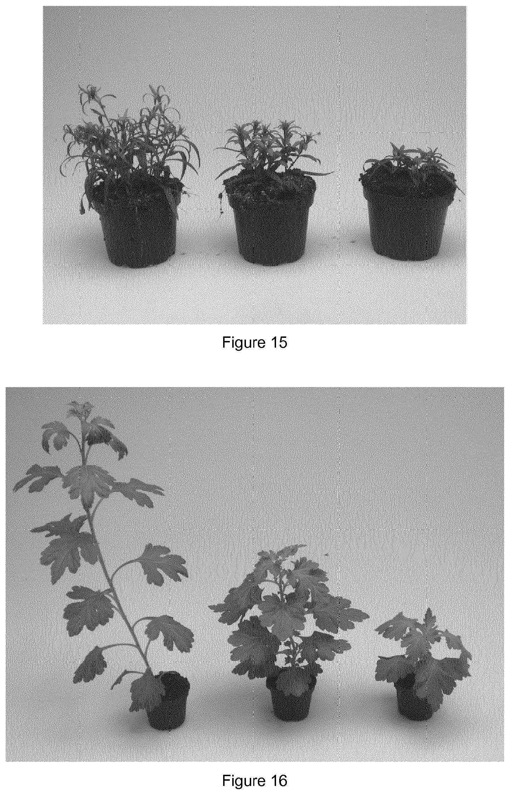

Figure 15 shows *Aster novi-belgii* '134A' wild type (left) and transformed with *Argobacterium rhizogenes* (centre and right), six weeks after start of vegetative propagation and two weeks into short day conditions for flower induction (light from 07:00 to 17:00). All plants were all pinched to the same height after four weeks. After 5 weeks the plant (centre) were sprayed to drop point with 0,5 mg GA3/L (ppm). The difference in flower induction between the wild type *Aster novi-belgii* '134A' (to the left) and the *rol* transformed *Aster novi-belgii* '134A' plant treated with GA3 (centre) is very clear. Plant growth and side shoot formation has increased in the GA3 treated and *rol* transformed *Aster novi-belgii* '134A' (centre), compared to untreated *rol* transformed *Aster novi-belgii* '134A' (to the right).
Figure 16 shows *Chrysantemum x morifolium* '2012-0047' wild type (left) and transformed with *Argobacterium rhizogenes* (centre and right), eight weeks after start of vegetative propagation and five weeks into short day conditions for flower induction (light from 07:00 to 17:00). After 5 weeks the plant (centre) was sprayed to drop point with 0,75 mg GA3/L (ppm). The difference in plant height and flower induction between the wild type *Chrysantemum x morifolium* '2012-0047' (to the left) and the *rol* transformed *Chrysantemum x morifolium* '2012-0047' plant treated with GA3 (centre) is very clear. Plant growth and side shoot formation has increased in the GA3 treated and *rol* transformed *Aster novi-belgii* '134A' (centre), compared to untreated *rol* transformed *Aster novi-belgii* '134A' (to the right).

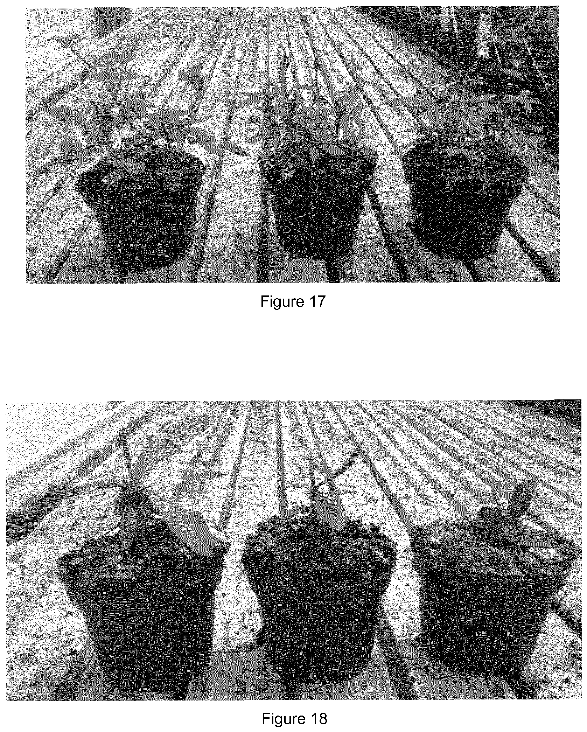

Figure 17 shows *Rosa hybrida* '2014-0004' wild type (left) and transformed with Argobacterium rhizogenes (centre and right), eight weeks after start of vegetative propagation and four weeks after pinching. All plants were pinched to the same height after four weeks. After 5 and 7 weeks the plant (centrer) was sprayed to drop point with 0,25 mg GA3/L (ppm). The difference in flower induction between the wild type *Rosa hybrida* '2014-0004' (to the left) and the *rol* transformed *Rosa hybrida* '2014-0004' plant treated with GA3 (centre) is very clear, flower buds are visible in the rol transformed and GA3 treated plant (centre). Internodes are clearly longest in wild type plant (right), intermediate in GA3 treated and *rol* transformed (centrr) and shortest in *rol* transformed *Rosa hybrida* '2014-0004' untreated control. Plant growth, flower and side shoot formation has increased in the GA3 treated and *rol* transformed *Rosa hybrida* '2014-0004' (centre), compared to untreated *rol* transformed *rol* transformed *Rosa hybrida* '2014-0004' (to the right).
Figure 18 shows *Euphorbia milii* '2014-0810' wild type (left) and transformed with Argobacterium rhizogenes (centre and right), eight weeks after start of vegetative propagation. After 4 and 6 weeks the plant (centre) was sprayed to drop point with 1,0 mg GA3/L (ppm). Internodes are clearly longest in wild type plant (right), intermediate in GA3 treated and *rol* transformed (centre) and shortest in *rol* transformed *Euphorbia milii* '2014-0810' untreated control.

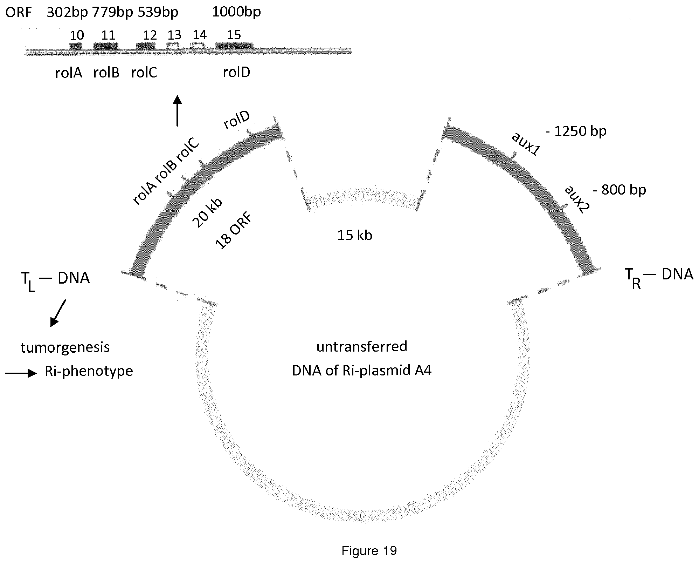

Figure 19 depicts an illustrative A. *rhizogenes* Ri-plasmid from an agropine strain. The T-DNA contains two segments, T_{L} and T_{R}, which are separated by a 15Kb sequence that is not integrated. The T_{L}-DNA contains 18 open reading frames (ORFs) where the four root loci-genes reside. The T_{R}-DNA contains several genes, including *aux1* and *aux2.*

### EXAMPLE 1: Transformation Materials and Methodology

### Plant material

*In vivo* plants of *Kalanchoe pinnata* (Knud Jepsen A/S, Hinnerup, Denmark and AgroTech a/s, Tåstrup, Denmark) were cultivated in a greenhouse with temperatures of 20°C at day and night, 16 hour day length and a light intensity of 260 µmol photons m⁻² s⁻¹*. In vitro plants* were cultivated in growth chamber with temperatures of 25°C at day and 22°C at night, 13-hour day length and a light intensity of 75 µmol photons m⁻² s⁻¹.

Leaf explants for each species/hybrid were used for control experiment. Leaves derived from *in vivo* material were sterilised in 70% EtOH for 1 min. followed by 20 min. in 1% NaOCI (VWR, Copenhagen, Denmark) and 0.03% (v/v) Tween 20 (Merck, La Jolla, USA) and washed 3 times in sterile water and were stored until excision.

### Bacterial strain

*Agrobacterium rhizogenes* strain ATCC43057 (A4) (kindly provided by Dr. Margareta Welander, Swedish University of Agricultural Sciences, Sweden) was used for induction of hairy roots. The strain was cultured in liquid MYA medium (Tepfer and Cassedelbart (1987) Microbiol Sci. 4, pp. 24-28. 1mL of the bacterial glycerol stock (kept at -80°C) was diluted in 10mL MYA in a 50mL Falcon tube and incubated for 8h at 27°C and shaken at 260 rpm. The solution was further diluted with 100mL MYA in a 250mL flask and shaken at 260 rpm for 24h in darkness at 27°C. The OD₆₀₀ = 0.4-0.6 was measured on Nanodrop 1000 (Thermo Scientific, Wilmington, DE, USA).

### Transformation

Sterilized leaves or *in vitro* plant were excised to pieces of min 1 cm x 1 cm and stored in sterile water until all explants were ready. The water was discarded from the explants and *A. rhizogenes*-suspension was added to cover all explants for 30 min. After 30 min. the *A. rhizogenes*-suspension was discarded and the slices were transferred, with a thin layer of the *A. rhizogenes* suspension on the surface, to co-cultivation plates for 24 h in darkness without selection. The explants were cultivated in the lab at temperatures at 22°C in darkness. After co-cultivation, the explants were transferred to 0-media (selection media) by drying the explants with pieces of ripped sterile filter paper. The leaf surface was as dry as possible on both sides of the excised leaf. The explants stayed in darkness until roots were developed enough to be transferred to regeneration media. The material was transformed over three sessions. The transformation was conducted with *K. pinnata* (see figure 1-4), *Kalanchoe gastonis-bonnieri* interspecific hybrid 'Tropical Parfait' (see figure 5), *Kalanchoe humilis* (see figure 6) and *Aster novo-belgii* (see figure 7). For each species, the controls and putative transformants was performed the same day.

### Basic medium

The basic medium used as background of all media used was ½ x MS (Sigma M0404) (consisting of Murashige and Skoog macro- and microelements) (Murashige and Skoog, 1962) at a concentration of 2,2 g L⁻¹, 30 g L⁻¹ sucrose (table sugar), 7g L⁻¹ bacto agar and 0,50 g L⁻¹ 2-(N-morpholino)-ethanesulphonic acid (MES) (Duchefa). The pH was adjusted to 6.3 by 1 M KOH and the media was autoclaved at 121°C and 103.5 kPa.

### Co-cultivation medium

Co-cultivation medium used for co-cultivation between explant and *A*. *rhizogenes* consisted of basic medium with 30 µg mL⁻¹ acetosyringone (Sigma-Aldrich, Steinheim, Germany).

### Selection medium

Selection medium was a hormone-free medium used for root formation of putatively transformed explants and controls. Filter-sterilized antibiotics were added after autoclaving to the selection media to the basic medium. Selection media consist of basic media ½ x MS medium with timentin (TIM) in the concentration of 100mg L⁻¹. Preferably, the selection medium contains arginine, preferably 0.5 mM arginine.

### Regeneration media

Regeneration medium containing the hormone N-(2-chloro-4-pyridyl)-N-phenylurea (CPPU) was used for regeneration of nodules on the putatively transformed root clusters. Filter-sterilised hormones and antibiotics were added after autoclaving to the regeneration media. The CPPU-medium contained basic ½ x MS medium with 1.5µg L⁻¹ CPPU together with TIM in the concentration 100mg L⁻¹.

### Co-cultivation

In all treatments the explants were co-cultivated for 24 hours. After co-cultivation, the explants were blotted onto sterile filter paper and thoroughly dried with ripped pieces of sterilised filter paper. Controls and putatively transformed explants were transferred to selection medium.

### Plant selection

After 24 hours of co-cultivation the explants were transferred to 0-media (selection medium) with 8 explants on each Petri dish. After several weeks, the increasing number of roots and decreasing number of explants (due to vitrification - the leaf sections became glass like or because of infections) were monitored for the specific Petri dish in the treatment.

### Plant regeneration

When the roots of putatively transformed explants had developed to a length of 1.5-2 cm they were transferred in clusters, with a part of the explant to CPPU-medium. The transferred root clusters were placed in a climate chamber (Celltherm, United Kingdom) on shelves with 11h daylight and day/night temperatures of 20/18°C and an intensity of 45-70µmol photons m⁻²s⁻¹ (Philips, Amsterdam, The Netherlands). Only root clusters with *A*. *rhizogenes* treated explants was transferred. Here the number of root clusters was monitored as well as the number of nodules developing from the roots. Counting of nodule development was stopped when no positive development was observed after 30 days for any of the four species.

### Control plants

Control plants were treated like transformants but inoculated in MYA medium without bacteria and with a lower number of explants-25 per cultivar. The control experiment plants were conducted in parallel with the transformants.

### Plant growth conditions

Plants described herein were grown in a greenhouse according to day length and temperatures as described in tables 1 and 2 below. The plants were produced in pots with a diameter of 10.5 cm or 13 cm. Cuttings were taken from vegetative (veg.) plants and grown and kept vegetative for the first 3-8 weeks following planting, depending on cultivar, species, genus, and pot size. The plants described in table 1 were transferred to flower inducing conditions 4-9 weeks after planting. Between 13-19 weeks after planting, depending on cultivar, species genus, pot size, and time of year, the plants entered their generative (gen.) stage - were mature with flowers that were opening or about to open.

The plants were grown under natural light conditions supplemented with 70 µmol photons m⁻² s⁻¹ SON-T light when the natural light was less than 100 µmol/m²/s. All plants, except *Phalaenopsis* and *Vanilla,* were grown in a peat based soil mix and were watered with a solution containing 200 parts per million (ppm) nitrogen, 200 ppm potassium, 40 ppm phosphorous, 200 ppm calcium, 40 ppm magnesium, 60 ppm sulphate, 1 ppm iron, 0.6 ppm manganese, 0.1 ppm copper, 0.1 ppm zinc, 0.3 ppm boron, 0.03 ppm molybdenum. For all plants, except *Phalaenopsis* and *Vanilla,* shading with curtains was active when light intensity was higher than 450 µmol photons m⁻² s⁻¹ and humidity was kept in the range between 60-80% relative humidity.

**Table 1: Growth conditions**

| | Light period (Max Light) | Dark period | Light temp | Night temp. | Genus |
|---|---|---|---|---|---|
| Short day | 07:00-17:00 | 17:00-07:00 | 19°C | 21°C | *Kalanchoe* (gen.) |
| | | | | | *Aster* (gen.) |
| | | | | | *Chrysanthemum* (gen.) |
| | | | | | *Euphorbia* (gen.) |
| | | | | | *Bouvardia* (gen.) |
| | | | | | *Rhodiola* (veg.) |
| Long day | 02:00-17:00 | 17:00-02:00 | 19°C | 21°C | *Kalanchoe* (veg.) |
| | | | | | *Aster* (veg.) |
| | | | | | *Chrysanthemum* (veg.) |
| | | | | | *Euphorbia* (veg.) |
| | | | | | *Bouvardia* (veg.) |
| | | | | | *Rhodiola* (gen.) |
| | | | | | *Strelitzia* |
| | | | | | *Hibiscus* |
| | | | | | *Mandevilla* |
| | | | | | *Echinacea* |
| | | | | | *Schisandra* |
| | | | | | *Rosa* |
| | | | | | *Ocimum* |
| | | | | | *Capsicum* |
| | | | | | *Ipomoea* |
| | | | | | *Solanum* |
| | | | | | *Nicotiana* |

*Phalaenopsis* and *Vanilla* were grown in a bark based soil mix and were watered with a solution containing 50 parts per million (ppm) nitrogen, 50 ppm potassium, 10 ppm phosphorous, 50 ppm calcium, 10 ppm magnesium, 15 ppm sulphate, 0.2 ppm iron, 0.1 ppm manganese, 0.01 ppm copper, 0.01 ppm zinc, 0.05 ppm boron, 0.005 ppm molybdenum.

*Vanilla* and *Phalaenopsis* plants were grown under natural light conditions. Shading with curtains was active when light intensity was higher than 250 µmol photons m⁻² s⁻¹ and humidity was keep in the range between 80-90% relative humidity.

**Table 2: Growth conditions for Vanilla and Phalaenopsis plants**

| | Light period | Dark period | Light temp | Night temp. |
|---|---|---|---|---|
| Vegetative growth before flowering | 06:00-18:00 | 18:00-06:00 | 24°C | 24°C |
| Cooling for flower induction | 06:00-18:00 | 18:00-06:00 | 14-17°C | 14-17°C |
| Generative growth after flower induction | 06:00-18:00 | 18:00-06:00 | 24°C | 24°C |

### Molecular analysis

DNA was isolated with DNeasy Plant Mini Kit (Qiagen, Hilden, Germany) from root clusters (and nodules) on regeneration medium. Half of a root cluster was harvested for DNA extraction from each of the genera/species. The concentration was measured on NanoDrop 1000 (Thermo Scientific, Wilmington, DE, USA). Concentrations were measured to 2 - 5 ng/µl. PCR on DNA was performed with a concentration of 15ng with the three specific primers (see Table 1 below) to amplify the *rol*B gene on the T_{L}-DNA and *KdActin* and *VirD2* as controls. Dimethyl sulfoxide (DMSO) was added to the PCR reaction to obtain a better unfolding of the DNA. The following temperature program was applied for amplification in the DNA thermal cycler (MyCycler, Biorad, Hercules, California, USA): 95°C for 10 min. (initial denaturation) followed by 40 cycles 95°C for 30 sec. (denaturation), 58°C for 30 sec. (annealing) and 72°C for 15 sec. (elongation), with a final 7 min. elongation at 72°C. The amplified fragments sequences were mixed with orange G (Sigma-Aldrich, SteinHeim, Germany) (40% sucrose (w/v) and 1.5% Orange G (w/v) and Gelred (Biotium, Hayward, CA, USA) and fractionated in 1% TAE agarose gel. Other rol genes or other gene sequences from *A. rhizogenes* in particular from the T_{L}- and T_{R}-DNA of the Ri plasmid can also be identified in a similar way, known to the skilled person.

**Table 3: Primer Sequences SEQ ID NOS 9-18, respectively, in order of appearance (Lütken et al.,Euphytica DOI 10.1007/s10681-012-0701-5.)**

| Gene | Primer sequence | Product size (bp) |
|---|---|---|
| *rol*A | 5'-CCAATCTGAGCACCACTCCT-3' | 153 |
| | 5'-AATCCCGTAGGTTTGTTTCG-3' | |
| *rol*B | 5'-GATATCCCGAGGGCATTTTT-3' | 182 |
| | 5'-GAATGCTTCATCGCCATTTT-3' | |
| *rol*C | 5'-CAATAGAGGGCTCAGGCAAG-3' | 202 |
| | 5'-CCTCACCAACTCACCAGGTT-3' | |
| *rol*D | 5'-GCGAAGTGGATGTCTTTGG-3' | 225 |
| | 5'-TTGCGAGGTACACTGGACTGA-3' | |
| *KdActin** | 5'-GCAGGACGTGATCTGACTGA-3' | 168 |
| | 5'-GACGGACGAGCTACTCTTGG-3' | |

### Statistical analysis

*K. pinnata* functioned as a reference of the transformation. Similarly, control explants had five replicates but 5 explants per species/hybrids with a total of 25 per species/hybrids. Since the explants may be taken out of the experiment because of infection, the number of explants changed over time. The total number of explants was therefore monitored to obtain a better ratio between number of explants and formation of roots. The number of roots was monitored as the number increased. The average of surviving explants per petri dish and the average of roots per petri dish were calculated. The two averages were used to calculate a ratio for each petri dish to describe the number of roots per explants.

Vₘₐₓ (root development/days) was modelled with a linear regression and using the slope. Standard deviations (SD) and students *t*-test (*t*-test) were calculated in Excel for each observation to verify variation within the individual species/hybrids. ANOVA test was performed with R (R is a free software environment for statistical computing).

### Gibberellin treatment

For gibberellin treatments, the product Berelex (9,6% gibberellic acid A3, GA3, Valent BioSciences, 870 Technology Way, Libertyville, IL, USA) or other GA3 based products was used. Said products were diluted 50,000 to 1,000,000 times. The plants were treated with 0.1 to 2.0 ppm Berelex (GA3) suspensions. Plants are e.g. grown in a greenhouse with a plant density ranging from 25 to 600 plants per m². The suspension was sprayed onto the plants with a volume ranging e.g. from 100 ml to 1 l per m² per treatment. 1 to 10 treatments were applied to the plants in their growing cycle, depending on the height of the plant as compared to the non-transformed control plant and the envisaged intermediate height to be obtained.

In addition to growth to intermediate height it was found that the flowering of rol transformed plants was affected by the application of gibberellic acid to the exterior of the plants.

It is quite well known by people skilled in the art of producing *Kalanchoe pinnata,* this species is challenging to trigger into flowering by short day treatment alone. It was now observed that rol transformed plants were responding more efficient to short day treatment than non-transformed control plants (Figure 3 and 4). After 5 weeks under short day conditions (light from 07:00 to 17:00) the plants were sprayed to drop point with 1,25 mg GA3/L (ppm) (from Berelex tablets, containing 9,6% gibberellic acid A3 isomer, Syngenta). This treatment was repeated after 7 weeks under short day conditions. In addition to plants growing to intermediate height, the difference in flower formation between the untreated rol transformed and the rol transformed plant treated with GA3 was very clear, in particular with regard to growing of the plants to intermediate height when mature. Further, flower formation and thus production time was significantly faster when rol transformed plants were treated with GA3. In *Kalanchoe pinnata,* flower formation appeared, to be absent or non-significant in nontransformed control plants. For other plants, reference is made to the above description of the figures.

### Results

The experiments involved a natural transformation with *Agrobacterium rhizogenes* to study the transformation efficiency for different species and hybrids and for plain material from *in vivo* and *in vitro.* The plants belonging to the species as described above were transformed with the conditions that were found optimal for *K. blossfeldiana* 'Molly' by Christensen *et al.,* (2008) or slightly changed for optimisation for each of the species. *K. blossfeldiana* 'Molly' was used as a control within the transformants since the cultivar formed background of the transformation system. Gibberellin treatment was performed as described above.

Root induction and growth were monitored as a total number of roots per petri dish in each treatment. Since some explants were removed due to infection the total number of explants over time was also monitored. This was done to obtain a more unbiased assessment when calculating the number of roots per explant in each plant line.

### Root development on 0-media

Root formation was found to take place in the following species; *Strelitzia reginae, Aster novi-belgii, Aster dumosus, Chrysantemum morifolium, Chrysantemum x morifolium* (syn. *C.* x *grandiflorum* e.g.*Dendranthema* hybrids, or hybrids between *Chrysantemum morifolium* and other *Chrysantemum* species e.g. *Chrysanthemum indicum, Euphorbia pulcherrima, Euphorbia milii, Bouvardia longiflora, Hibiscus rosa-sinensis, Hibiscus schizopetalus, Hibiscus sabdariffa, Hibiscus syriacus, Hibiscus trionum, Hibiscus cannabinus, Mandevilla* × *amabilis, Mandevilla sanderi, Mandevilla splendens, Nicotiana tabacum, Nicotiana sylvestris, Nicotiana x sanderrae, Phalaenopsis amabilis, Phalaenopsis amboinensis, Phalaenopsis aphrodite, Phalaenopsis appendiculata, Vanilla planifolia, Ocimum basilicum, Capsicum annuum, Capsicum baccatum, Capsicum chinense, Capsicum frutescens, Capsicum pubescens, Ipomoea batatas, Solanum lycopersicum, Solanum tuberosum, Solanum nicotiana, Echinacea purpurea, Echinacea angustifolia, Echinacea pallida, Rosa hybrida, Rosa canina, Rosa spinosissima, Rosa damascena "trigintipetala', Rosa centifolia, Schisandra chinensis, Schisandra glabra, Schisandra rubriflora, Rhodiola rosea, Kalanchoe pinnata, Kalanchoe marmorata, Kalanchoe gastonis-bonnieri, Kalanchoe dixoniana, Kalanchoe humilis, Kalanchoe laciniata,* and was transferred to regeneration medium no later than 100 days After transfer to 0-media. At the time of the first time of transfer to regeneration medium putative transformants from *Strelitzia reginae, Aster novi-belgii, Aster dumosus, Chrysantemum morifolium, Chrysantemum x morifolium* (syn. *C*. x *grandiflorum* e.g.*Dendranthema* hybrids, or hybrids between *Chrysantemum morifolium* and other *Chrysantemum* species e.g. *Chrysanthemum indicum, Euphorbia pulcherrima, Euphorbia milii, Bouvardia longiflora, Hibiscus rosa-sinensis, Hibiscus schizopetalus, Hibiscus sabdariffa, Hibiscus syriacus, Hibiscus trionum, Hibiscus cannabinus, Mandevilla* × *amabilis, Mandevilla sanderi, Mandevilla splendens, Nicotiana tabacum, Nicotiana sylvestris, Nicotiana x sanderrae Phalaenopsis amabilis, Phalaenopsis amboinensis, Phalaenopsis aphrodite, Phalaenopsis appendiculata, Vanilla planifolia, Ocimum basilicum, Capsicum annuum, Capsicum baccatum, Capsicum chinense, Capsicum frutescens, Capsicum pubescens, Ipomoea batatas, Solanum lycopersicum, Solanum tuberosum, Solanum nicotiana, Echinacea purpurea, Echinacea angustifolia, Echinacea pallida, Rosa hybrida, Rosa canina, Rosa spinosissima, Rosa damascena "trigintipetala', Rosa centifolia, Schisandra chinensis, Schisandra glabra, Schisandra rubriflora, Rhodiola rosea, Kalanchoe pinnata, Kalanchoe marmorata, Kalanchoe gastonis-bonnieri, Kalanchoe dixoniana, Kalanchoe humilis, Kalanchoe laciniata,* were significantly different from control.

### Possible use of transformed and gibberellin treated plants according to the invention

### Kalanchoe

### Examples:

- *Kalanchoe pinnata*
- *Kalanchoe marmorata*
- *Kalanchoe gastonis-bonnieri interspefic hybrid 'Tropical Parfait'*
- *Kalanchoe dixoniana*
- *Kalanchoe humilis*
- *Kalanchoe laciniata interspecific hybrid 'Amazing Pink'*

Current use primarily as an ornamental plant. An intermediate plant height and increased branching was observed as compared to non-transformed and non-gibberellin treated plants. Also, content of secondary metabolites e.g. bryophillin A, showing strong anti-tumour promoting activity does make this genus/species interesting as a medicinal plant. *Kalanchoe,* in particular *K. pinnata* also contains -coumaric acid, Ferulic acid, Syringic acid, Caffeic acid, citric acid, isocitric acid, malic acid, P-hydroxybenzoic acid, Flavnoids as quercetin, kaempferol, Quercetin-3-diarabinoside, Kaempferol-3-glucoside, Quercetin-3-L-rhamnosido-L-arabino furanoside, η-hentricontane, η-tritriacontane, Sitosterol. Studies showed that several of these compounds exhibited higher concentrations in *rol* transformed plants.

Any of these compounds can be isolated from *Kalanchoe* and used as or in a human or animal necessity such as a medicament. Plants transformed with the Ri plasmid of *A. rhizogenes* comprising the *rol* genes and treated with gibberellin exhibited higher concentrations compared to control.

### Rosa

### Examples:

- *Rosa hybrida*
- *Rosa canina*
- *Rosa spinosissima*
- *Rosa damascena 'Trigintipetala'*
- *Rosa centifolia*
Current use primarily as an ornamental plant. An intermediate plant height and increased branching is observed as compared to non-transformed controls, making it possible to have sufficient branching from fewer cuttings per pot. Rosa is also having significant use in the perfume industry. In Europe, *Rosa damascena 'Trigintipetala'* is particularly used, and *Rosa centifolia* in other parts of the world. The main constituents are the fragrant alcohols geraniol and I-citronellol and rose camphor. β-Damascenone is also a significant contributor to the scent. Plants transformed with the Ri plasmid of *A. rhizogenes* comprising the *rol* genes and treated with gibberellin exhibited higher concentrations compared to control.

### Strelitzia

### Example:

- *Strelitzia reginae*
Mainly used as a cut flower because of its size. A significant reduction in plant height to intermediate value and also significantly in production time (time to flowering) is observed in plants, transformed with *Agrobacterium rhizogenes* comprising the Ri plasmid and treated with gibberellin.

### Aster

### Example:

- *Aster novi-belgii*
- *Aster dumosus*
Mainly used as potted plant and cut flower. An intermediate plant height and increased branching is observed as compared to non-transformed non gibberellin treated controls, making it possible to have sufficient branching from fewer cuttings per pot.

### Chrysantemum

### Examples:

- *Chrysanthemum morifolium*
- *Chrysanthemum indicum*
- *Chrysanthemum* × *morifolium (Dendranthema hybrids)*

Mainly used as potted plant and cut flower, but also as ingredient in Chrysanthemum tea (*Chrysanthemum indicum*)*.* Intermediate plant height and increased branching was observed in transformants making it possible to have sufficient branching from fewer cuttings per pot when using the plants as potted plants. Flavour of tea extracts made from Ri-transformed *Chrysanthemum indicum* and *Chrysanthemum morifolium* appeared to be more intense than that of not transformed and not gibberellin treated counterparts.

### Euphorbia

### Example:

- *Euphorbia milii*
- *Euphorbia pulcherrima* (Poinsettia)
Mainly used as an ornamental plant. An intermediate plant height and increased branching was observed as compared to non-transformed non gibberellin treated plants, making it possible to have sufficient branching without pinching and from fewer cuttings per pot.

### Hibiscus

### Examples

- *Hibiscus rosa-sinensis*
- *Hibiscus schizopetalus*
- *Hibiscus sabdariffa*
- *Hibiscus syriacus*
- *Hibiscus trionum*
- *Hibiscus cannabinus*
Mainly used as potted plant or ornamental garden plants in temperate areas (*Hibiscus syriacus*) and in the tropics/subtropics (all species). Intermediate plant height and increased branching is observed in transformants making it possible to have sufficient branching without pinching and from fewer cuttings per pot. *Hibiscus* flowers contain anthocyanins, and plants transformed with the Ri plasmid of *A. rhizogenes* comprising the *rol* genes and treated with gibberellin exhibited higher concentrations as compared to control.

### Dipladenia/Mandevilla

### Examples:

- *Mandevilla* ×*amabilis*
- *Mandevilla sanderi*
- *Mandevilla splendens*
Mainly used as potted plant or ornamental garden plants in tropics/subtropics. In transformants, intermediate plant height and increased branching was observed making it possible to have sufficient branching without pinching and from fewer cuttings per pot. Costly work attaching the plants to a physical fixture will not be needed in *rol* transformants, treated with gibberellin.

### Nicotiana

### Examples:

- *Nicotiana tabacum* Mainly used as crop plants for the production of tobacco. Tobacco leaves normally contain 2 to 8% nicotine and plants, transformed with the Ri plasmid of *A. rhizogenes* comprising the *rol* genes exhibited higher concentrations compared to control.
- *Nicotiana sylvestris*
- *Nicotiana x sanderrae,*
Mainly used as ornamental/bedding plants. An intermediate plant height and increased branching was observed making it possible to have sufficient branching without pinching and from fewer cuttings per pot.

### Bouvardia

### Example:

- *Bouvardia longiflora*
Mainly used as potted plant and cut flower. In transformed plants, an intermediate plant height and increased branching is observed making it possible to have sufficient branching from fewer cuttings per pot with less chemical growth regulation needed.

### Phalaenopsis

### Example:

- *Phalaenopsis amabilis*
- *Phalaenopsis amboinensis*
- *Phalaenopsis Aphrodite*
- *Phalaenopsis appendiculata*
Used as potted plant and cut flower, the largest potted plant product (produced numbers and turnover) in Europe. An intermediate plant height and increased branching was observed in transformed plants, treated with gibberellin, making it possible to have more spikes per plant using less chemical growth regulation with expected higher prices on the market.

### Vanilla planifolia

*Vanilla* is one of the primary sources for vanilla flavouring, due to its high vanillin content (4-hydroxy-3-methoxybenzaldehyde) and plants, transformed with the Ri plasmid of *A. rhizogenes* comprising the *rol* genes and treated with gibberellin exhibited intermediate growth and higher concentrations of vanillin compared to control.

### Basil

### Example:

- *Ocimum basilicum*
Mainly used as an edible plant. The leaves may taste somewhat like anise, with a strong, pungent, often sweet smell, due to its content of metylchavicol, kineol and linalool. Plants transformed with the Ri plasmid of *A. rhizogenes* comprising the rol genes and treated with gibberellin exhibited intermediate height and contained higher concentrations compared to control.

### Capsicum

### Examples:

- *Capsicum annuum,*
- *Capsicum baccatum,*
- *Capsicum chinense,*
- *Capsicum frutescens,*
- *Capsicum pubescens.*
Mainly used as spices and food vegetables, but *Capsicum* containing capsaicin (methyl vanillyl nonenamide), has also found use in medicines to stimulate blood circulation or to relieve pain. Plants transformed with the Ri plasmid of *A. rhizogenes* comprising the *rol* genes and treated with gibberellin exhibited intermediate height and contained higher concentrations capsaicin compared to control.

### Ipomoea

### Example:

- *Ipomoea batatas*
Known as sweet potato. Its large, starchy, sweet-tasting, tuberous roots are a root vegetable. Besides starches, sweet potatoes are rich in complex carbohydrates, dietary fibre and in beta-carotene (a provitamin A carotenoid), while having moderate contents of other minerals and vitamins, including vitamin B5, vitamin B6, manganese and potassium. Plants transformed with the Ri plasmid of *A. rhizogenes* comprising the *rol* genes and treated with gibberellin exhibited intermediate height and contained higher concentrations of the minerals and vitamins compared to control.

### Tomato

### Example:

- *Solanum lycopersicum*
The tomato is the edible, often red/orange/yellow/greenish fruit/berry. The fruit contains lycopene, a powerful antioxidant, has been linked with reduced risks of colorectal, gastric, lung, prostate, and pancreas cancer. Intermediate plant height and increased branching was observed, making it possible to use the plant also as a houseplant with edible fruits or in gardens with limited space available. Furthermore, it was noticed that content of lycopene increased in fruits of plants, transformed with the Ri plasmid of *A. rhizogenes* comprising the *rol* genes and treated with gibberellin.

### Potato

### Example

- *Solanum tuberosum*
The potato is and edible plant, containing vitamins and minerals, as well as an assortment of phytochemicals, such as carotenoids and natural phenols.

Intermediate plant height and increased branching was observed making it possible to use the plant also as a garden plant with edible tubers in gardens with limited space available Furthermore it was noticed that content of vitamin and mineral content increased in tuber of *rol* transformed potato plants, treated with gibberellin. Also, a more intense taste was observed.

### Solanum nicotianum

*Solanum nicotianum* is an intergeneric graft chimera of *Nicotiana tabacum L.* and *Solanum laciniatum* (Kaddoura, R.L. and Mantell, S.H., (1991) Ann. Bot. 68 (6): 547-556, used as an ornamental plant. An intermediate plant height and increased branching is observed as compared to non-transformed and non-gibberellin treated controls, making it possible to have sufficient branching from fewer cuttings per pot.

### Echinacea

### Examples:

- *Echinacea purpurea*
- *Echinacea angustifolia,*
- *Echinacea pallida*
*Echinacea* is mainly used for herbal medicines - containing phenyl propanoid, echinacoside. The constituent base for Echinacea is complex, consisting of a wide variety of chemicals of variable effect and potency. The range of active substances have antimicrobial, stimulating or modulating effects on different parts of the immune system. All species contain phenols, phenyl propanoid constituents such as cichoric acid and caftaric acid are present in *E. purpurea,* other phenols include echinacoside. Other chemical constituents that may be important in echinacea health effects include alkylamides and polysaccharides. The immunomodulatory effects of echinacea preparations are likely caused by fat-soluble alkylamides (alkamides). Alkylamides have similar potency to that of THC at the CB2 receptor, with THC being around 1.5 times stronger (∼40 nm vs ∼60 nm affinities). However, potency is dramatically less than that of THC at the psychoactive CB1 receptor (∼40 nm vs ∼ >1500 nm affinities). It was now observed that concentration of a range of chemical content increased in parts of plants, transformed with the Ri plasmid of *A. rhizogenes* comprising the *rol* genes and treated with gibberellin. Furthermore, intermediate plant height and increased branching was observed making it possible to use the plant also as a house/garden plant with ornamental value.

### Schisandra

### Examples:

- *Schisandra chinensis*
- *Schisandra glabra*
- *Schisandra rubriflora (ornamental use)*

The berries of *Schisandra* are used in traditional Chinese medicine. Chemical constituents include the lignans schisandrin, deoxyschisandrin, gomisins, and pregomisin, which are found in the seeds of the fruit. It was observed that concentration of a range of chemical content increased plant parts of plants, transformed with the Ri plasmid of *A. rhizogenes* comprising the *rol* genes and treated with gibberellin. Furthermore, intermediate plant height and increased branching was observed, making it possible to use the plant also as a house/garden plant with ornamental value.

### Rhodiola

### Example:

### • Rhodiola rosea

*Rhodiola* has been used in herbal medicine in China, Russia, and Scandinavia to better cope with the cold Siberian weather. The aerial portion is consumed as food in some parts of the world, sometimes added to salads. The root and other plant parts contain rosavin, rosarin, rosin and salidroside (and sometimes p-tyrosol, rhodioniside, rhodiolin and rosiridin. In addition to intermediate height, it was observed that the concentration of a range of chemical contents increased in plant parts of *rol* transformed plants, treated with gibberellin. The plant is quite slow growing, and the ornamental value of the plant is limited.

## Claims

1. Method for obtaining a plant having a height of 50 - 75% of the height of a corresponding wild type plant, and not less than 80% of the number of flowers thereof, comprising the steps of:
(a) transforming tissue of the wild type plant with one or more genes originating from the Ri plasmid of *Agrobacterium rhizogenes,*
(b) allowing the transformed tissue to develop roots,
(c) selecting a putatively transformed root having a hairy root phenotype;
(d) growing the selected root on a regeneration medium;
(e) allowing a transformed rooted plantlet to generate from said root;
(f) growing said transformed rooted plantlet into a mature transformed mother plant having a height of less than 40% of that of the corresponding wild type plant;
(g) preparing a transformed cutting from the mature transformed mother plant,
(h) allowing a rooted transformed plantlet to develop from the transformed cutting,
(i) treating the rooted transformed plantlet with gibberellin, therewith allowing the plantlet to develop in a mature plant having a height between the height of the transformed mother plant of step (f) and the height of the wild type plant of step (a).

2. Method of claim 1, wherein step (a) comprises:
- co-cultivating *A. rhizogenes* with a plant or plant part, allowing *A. rhizogenes* to deliver the said one or more genes of the Ri plasmid into said plant or plant part, and/or
- infection with *Agrobacterium rhizogenes,* preferably being wild-type *Agrobacterium rhizogenes.*

3. Method of claim 1 or 2, wherein the one or more genes originating from the Ri plasmid of *Agrobacterium rhizogenes* comprises one or more *rol* genes.

4. Method according to any of the preceding claims, wherein the mature transformed mother plant of step (f) has a height of less than 30%, preferably less than 25% of the height of the wild type plant of step (a).

5. Method according to any of the preceding claims, step (f) further comprising:
- treating the transformed rooted plantlet with gibberellin before the transformed rooted plantlet reaches maturity, and/or
- assaying the presence of one or more genes originating from the Ri plasmid of *Agrobacterium rhizogenes* in said mature transformed mother plant.

6. Method according to any of the preceding claims, wherein step (i) comprises:
- repeating treatment with gibberellin before the transformed plantlet reaches maturity, and/or
- allowing the plantlet to develop in a mature plant having a height of up to 70%, preferably up to 65% of the height of the wild type plant of step (a).

7. Method according to any of the preceding claims, wherein the wild type plant of step (a) belongs to any of the following genera:
- *Kalanchoe,* in particular K. *blossfeldiana, K. laciniata, K. pinnata, K. marmorata, K. gastonis-bonnieri, K. dixoniana, K. humilis, K. ambolensis, K. aromatica, K. campanulata, K. citrina, K. coccinea, K. crundallii, K. daigremontiana, K. decumbens, K. faustii, K. fedtschenkoi, K. figueredoi, K. flammea, K. glaucescens, K. gracilipes, K. grandiflora, K. guignardii, K. jongmansii, K. laciniata, K.latisepela, K. laxiflora, K. lobata, K. longiflora, K. manginii, K. nyikae, K. obtuse, K. paniculata, K. porphyrocalyx, K. prittwitzii, K. pubescens, K. pumila, K. rauhii, K. rotundifolia, K. scapigera, K. schumacherii, K. spathulata, K. streptantha, K. synsepala, K. tomentosa, K. thyrsiflora, K. tubiflora, K. uniflora;*
- *Chrysantemum,* in particular *Chrysantemum morifolium, Chrysantemum x morifolium (syn. C. x grandiflorum e.g.Dendranthema hybrids, or hybrids between Chrysantemum morifolium* and other *Chrysantemum* species, such as *Chrysanthemum indicum;*
- *Aster,* in particular *Aster novi-belgii, Aster dumosus;*
- *Rosa,* in particular *Rosa hybrida, Rosa canina, Rosa spinosissima, Rosa damascena "trigintipetala', Rosa centifolia;*
- *Solanum,* in particular *Solanum lycopersicum, Solanum tuberosum, Solanum nicotiana;*
- *Euphorbia,* in particular *Euphorbia pulcherrima, Euphorbia milii;*
- *Phaelanopsis,* in particular *Phalaenopsis amabilis, Phalaenopsis amboinensis, Phalaenopsis aphrodite, Phalaenopsis appendiculata;*
- *Ocimum,* in particular *Ocimum basilicum;*
- *Capsicum,* in particular *Capsicum annuum, Capsicum baccatum, Capsicum chinense, Capsicum frutescens, Capsicum pubescens;*
- *Mentha,* in particular *Mentha arvensis, Mentha requienii, Mentha spicata, Mentha longifolia, Mentha pulegium, Mentha suaveolens, Mentha aquatic, Mentha arvensis x spicata, Mentha aquatica x arvensis, Mentha x piperita;*
- *Hibiscus,* in particular *Hibiscus rosa-sinensis, Hibiscus schizopetalus, - Hibiscus sabdariffa, Hibiscus syriacus, Hibiscus trionum, Hibiscus cannabinus;*
- *Mandevilla*/*Dipladenia,* in particular *Mandevilla* ×*amabilis, Mandevilla sanderi, Mandevilla splendens;*
- *Eustoma,* in particular *Eustoma russellianum, Eustoma exaltatum;*
- *Lavendula,* in *particular Lavandula angustifolia, Lavandula; latifolia,Lavandula lanata,Lavandula dentate, Lavandula stoechas, Lavandula pedunculata, Lavandula viridis;*
- *Lillium,* in particular *Lilium bolanderi, Lilium* × *burbankii, Lilium canadense, Lilium columbianum, Lilium grayi, Lilium humboldtii, Lilium kelleyanum, Lilium kelloggii, Lilium maritimum, Lilium michauxii, Lilium michiganense, Lilium occidentale, Lilium* × *pardaboldtii, Lilium pardalinum, Lilium parryi, Lilium parvum, Lilium philadelphicum, Lilium pitkinense, Lilium superbum, Lilium ollmeri, Lilium washingtonianum, Lilium wigginsii;*
- *Clematis,* in particular *Clematis addisonii, Clematis albicoma, Clematis alpine, Clematis aristata, Clematis armandii, Clematis baldwinii,Clematis bigelovii, Clematis brachiate, Clematis campaniflora, Clematis catesbyana, Clematis chinensis, Clematis chrysocoma, Clematis cirrhosa, Clematis coactilis, Clematis Columbiana, Clematis crispa, Clematis dioica, Clematis drummondii , Clematis durandii, Clematis ispahanica, Clematis fawcettii, Clematis flammula, Clematis florida, Clematis fremontii, Clematis glaucophylla, Clematis glycinoides, Clematis henryi Clematis hirsutissima, Clematis integrifolia, Clematis* × *jackmanii, Clematis lanuginose, Clematis lasiantha, Clematis leptophylla, Clematis ligusticifolia, Clematis macropetala, Clematis marmoraria, Clematis microphylla, Clematis montana, Clematis morefieldii, Clematis napaulensis, Clematis occidentalis, Clematis ochroleuca, Clematis orientalis, Clematis palmeri, Clematis, Clematis patens, Clematis pauciflora, Clematis pickeringii, Clematis pitcher, Clematis recta, Clematis reticulate, Clematis rhodocarpa, Clematis smilacifolia, Clematis socialis, Clematis stans, Clematis tangutica, Clematis terniflora, Clematis ternifolia, Clematis texensis, Clematis versicolor, Clematis viorna, Clematis virginiana, Clematis vitalba, Clematis viticaulis, Clematis viticella;- Geranium,* in particular *Geranium cinereum, Geranium clarkei, Geranium dalmaticum, Geranium endressii, Geranium fremontii, Geranium himalayense, Geranium ibericum, Geranium macrorrhizum, Geranium maculatum, Geranium maderense, Geranium* × *magnificum, Geranium phaeum, Geranium platypetalum, Geranium pretense, Geranium psilostemon, Geranium renardii, Geranium sanguineum, Geranium subcaulescens, Geranium sylvaticum;*
- *Pelargonium,* in particular *Pelargonium crispum, Pelargonium grandiflorum, Pelargonium peltatum, Pelargonium graveolens, Pelargonium denticulatum, Pelargonium x hortorum,, Pelargonium x asperum;*
- *Nicotiana,* in particular *Nicotiana tabacum, Nicotiana sylvestris, Nicotiana x sanderrae;*
- *Bouvardia,* in particular *Bouvardia longiflora;*
- *Vanilla,* in particular *Vanilla planifolia;*
- *Ipomoea,* in particular *Ipomoea batatas;*
- *Echinacea,* in *particular Echinacea purpurea, Echinacea angustifolia, Echinacea pallida;*
- *Schisandra,* in particular *Schisandra chinensis, Schisandra glabra, Schisandra rubriflora;*
- *Rhodiola,* in particular *Rhodiola rosea;*
- *Leucanthemum,* in particular *Leucanthemum maximum, Leucanthemum paludosum, Leucanthemum x superbum, Leucanthemum vulgare, Leucanthemum adustum, Leucanthemum graminifolium, Leucanthemum integrifolium, Leucanthemum lacustre, Leucanthemum monspeliense, Leucanthemum pallens, Leucanthemum praecox, Leucanthemum subglaucum, Leucanthemum sylvaticum, Leucanthemum waldsteinii;*
- *Strelitzia,* in particular *Strelitzia reginae,*
including interspecific hybrids within the genera, and progeny thereof.

8. Method of any of the preceding claims, step (f) further comprising selecting a mature transformed mother plant, having not less than 80%, preferably not less than 90%, more preferably not less than 100% of the branching as compared to the wild type plant of step (a) from which a transformed cutting is prepared in step (g).

9. Method of any of the preceding claims, wherein the obtained plant has not less than 90% of the number of flowers as compared to the wild type plant of step (a).

10. Method of any of the preceding claims, wherein the obtained plant has not less than 100% of the number of flowers as compared to a not transformed control plant.

11. Method of any of the preceding claims, wherein the obtained plant does not have a delayed flowering time by more than 4 days, preferably by more than 2 days, as compared to the wild type plant of step (a).

12. Plant obtained by the method according to any of the preceding claims, comprising one or more genes originating from the Ri plasmid of *Agrobacterium rhizogenes.*

## Patentansprüche

1. Verfahren zum Erhalten einer Pflanze mit einer Höhe von 50 - 75% der Höhe einer entsprechenden Wildtyp-Pflanze und nicht weniger als 80% der Anzahl ihrer Blüten, umfassend die folgenden Schritte:
(a) Transformieren von Gewebe der Wildtyp-Pflanze mit einem oder mehreren Genen, die aus dem Ri-Plasmid von *Agrobacterium rhizogenes* stammen,
(b) dem transformierten Gewebe die Entwicklung von Wurzeln zu ermöglichen,
(c) Auswahl einer vermeintlich transformierten Wurzel mit einem behaarten Wurzelphänotyp;
(d) Züchten der ausgewählten Wurzel auf einem Regenerationsmedium;
(e) Ermöglichen der Bildung eines transformierten bewurzelten Pflänzchens aus dieser Wurzel;
(f) Züchten des transformierten, bewurzelten Pflänzchens zu einer reifen, transformierten Mutterpflanze mit einer Höhe von weniger als 40% der Höhe der entsprechenden Wildtyp-Pflanze;
(g) Vorbereitung eines transformierten Stecklings aus der reifen transformierten Mutterpflanze,
(h) aus dem transformierten Steckling ein verwurzeltes, transformiertes Pflänzchen entstehen zu lassen,
(i) Behandlung des bewurzelten transformierten Pflänzchens mit Gibberellin, wodurch sich das Pflänzchen in einer reifen Pflanze mit einer Höhe zwischen der Höhe der transformierten Mutterpflanze der Stufe (f) und der Höhe der Wildtyp-Pflanze der Stufe (a) entwickeln kann.

2. Verfahren nach Anspruch 1, wobei Schritt (a) umfasst:
- die Co-Kultivierung von A. *rhizogenes* mit einer Pflanze oder einem Pflanzenteil, wobei es A. *rhizogenes* ermöglicht wird, das genannte eine oder mehrere Gene des Ri-Plasmids in die genannte Pflanze oder den genannten Pflanzenteil einzubringen, und/oder
- Infektion mit *Agrobacterium rhizogenes,* vorzugsweise mit dem Wildtyp *Agrobacterium rhizogenes.*

3. Verfahren nach Anspruch 1 oder 2, wobei das eine oder die mehreren Gene, die aus dem Ri-Plasmid des *Agrobacterium rhizogenes* stammen, ein oder mehrere *rol*-Gene umfassen.

4. Verfahren nach einem der vorherigen Ansprüche, wobei die reife transformierte Mutterpflanze aus Schritt (f) eine Höhe von weniger als 30%, vorzugsweise weniger als 25% der Höhe der Wildtyp-Pflanze aus Schritt (a) aufweist.

5. Verfahren nach einem der vorherigen Ansprüche, wobei Schritt (f) ferner umfaßt:
- Behandlung des transformierten bewurzelten Pflänzchens mit Gibberellin, bevor das transformierte bewurzelte Pflänzchen die Reife erreicht, und/oder
- Bestimmung des Vorhandenseins eines oder mehrerer Gene, die aus dem Ri-Plasmid von *Agrobacterium rhizogenes* stammen, in der reifen transformierten Mutterpflanze.

6. Verfahren nach einem der vorherigen Ansprüche, wobei Schritt (i) umfaßt:
- Wiederholen der Behandlung mit Gibberellin, bevor das transformierte Pflänzchen die Reife erreicht, und/oder
- es dem Pflänzchen ermöglichen, sich in einer reifen Pflanze mit einer Höhe von bis zu 70%, vorzugsweise bis zu 65% der Höhe der Wildtyp-Pflanze der Stufe (a) zu entwickeln.

7. Verfahren nach einem der vorherigen Ansprüche, wobei die Wildtyp-Pflanze der Stufe (a) zu einer der folgenden Gattungen gehört:
- *Kalanchoe,* insbesondere *K*. *blossfeldiana, K. laciniata, K. pinnata, K. marmorata, K. gastonis-bonnieri, K. dixoniana, K. humilis, K. ambolensis, K. aromatica, K. campanulata, K. citrina, K. coccinea, K. crundallii, K. daigremontiana, K. decumbens, K. faustii, K. fedtschenkoi, K. figueredoi, K. flammea, K. glaucescens, K. gracilipes, K. grandiflora, K. guignardii, K. jongmansii, K. laciniata, K.latisepela, K. laxiflora, K. lobata, K. longiflora, K. manginii, K. nyikae, K. obtuse, K. paniculata, K. porphyrocalyx, K. prittwitzii, K. pubescens, K. pumila, K. rauhii, K. rotundifolia, K. scapigera, K. schumacherii, K. spathulata, K. streptantha, K. synsepala, K. tomentosa, K. thyrsiflora, K. tubiflora, K. uniflora;*
- *Chrysantemum,* insbesondere *Chrysantemum morifolium, Chrysantemum x morifolium (syn. C. x grandiflorum* z.B. *Dendranthema* Hybride, oder Hybride zwischen *Chrysantemum morifolium* und anderen *Chrysantemum*-Arten, wie *Chrysanthemum indicum;*
- *Aster,* insbesondere *Aster novi-belgii, Aster dumosus;*
- Rosa, insbesondere *Rosa hybrida, Rosa canina, Rosa spinosissima, Rosa damascena "trigintipetala', Rosa centifolia;*
- *Solanum,* insbesondere *Solanum lycopersicum, Solanum tuberosum, Solanum nicotiana;*
- *Euphorbia,* insbesondere *Euphorbia pulcherrima, Euphorbia milii;*
- *Phaelanopsis,* insbesondere *Phalaenopsis amabilis, Phalaenopsis amboinensis, Phalaenopsis aphrodite, Phalaenopsis appendiculata;*
- *Ocimum,* insbesondere *Ocimum basilicum;*
- *Capsicum,* insbesondere *Capsicum annuum, Capsicum baccatum, Capsicum chinense, Capsicum frutescens, Capsicum pubescens;*
- *Mentha,* insbesondere *Mentha arvensis, Mentha requienii, Mentha spicata, Mentha longifolia, Mentha pulegium, Mentha suaveolens, Mentha aquatic, Mentha arvensis x spicata, Mentha aquatica x arvensis, Mentha x piperita;*
- *Hibiscus,* insbesondere *Hibiscus rosa-sinensis, Hibiscus schizopetalus, Hibiscus sabdariffa, Hibiscus syriacus, Hibiscus trionum, Hibiscus cannabinus;*
- *Mandevilla*/*Dipladenia,* insbesondere *Mandevilla* ×*amabilis, Mandevilla sanderi, Mandevilla splendens;*
- *Eustoma,* insbesondere *Eustoma russellianum, Eustoma exaltatum;*
- *Lavendula,* insbesondere *Lavandula angustifolia, Lavandula; latifolia,Lavandula lanata,Lavandula dentate, Lavandula stoechas, Lavandula pedunculata, Lavandula viridis;*
- *Lillium,* insbesondere *Lilium bolanderi, Lilium* × *burbankii, Lilium canadense, Lilium columbianum, Lilium grayi, Lilium humboldtii, Lilium kelleyanum, Lilium kelloggii, Lilium maritimum, Lilium michauxii, Lilium michiganense, Lilium occidentale, Lilium* × *pardaboldtii, Lilium pardalinum, Lilium parryi, Lilium parvum, Lilium philadelphicum, Lilium pitkinense, Lilium superbum, Lilium ollmeri, Lilium washingtonianum, Lilium wigginsii;*
- *Clematis,* insbesondere *Clematis addisonii, Clematis albicoma, Clematis alpine, Clematis aristata, Clematis armandii, Clematis baldwinii,Clematis bigelovii, Clematis brachiate, Clematis campaniflora, Clematis catesbyana, Clematis chinensis, Clematis chrysocoma, Clematis cirrhosa, Clematis coactilis, Clematis Columbiana, Clematis crispa, Clematis dioica, Clematis drummondii, Clematis durandii, Clematis ispahanica, Clematis fawcettii, Clematis flammula, Clematis florida, Clematis fremontii, Clematis glaucophylla, Clematis glycinoides, Clematis henryi Clematis hirsutissima, Clematis integrifolia, Clematis* × *jackmanii, Clematis lanuginose, Clematis lasiantha, Clematis leptophylla, Clematis ligusticifolia, Clematis macropetala, Clematis marmoraria, Clematis microphylla, Clematis montana, Clematis morefieldii, Clematis napaulensis, Clematis occidentalis, Clematis ochroleuca, Clematis orientalis, Clematis palmeri, Clematis, Clematis patens, Clematis pauciflora, Clematis pickeringii, Clematis pitcher, Clematis recta, Clematis reticulate, Clematis rhodocarpa, Clematis smilacifolia, Clematis socialis, Clematis stans, Clematis tangutica, Clematis terniflora, Clematis ternifolia, Clematis texensis, Clematis versicolor, Clematis viorna, Clematis virginiana, Clematis vitalba, Clematis viticaulis, Clematis viticella;*
- *Geranium,* insbesondere *Geranium cinereum, Geranium clarkei, Geranium dalmaticum, Geranium endressii, Geranium fremontii, Geranium himalayense, Geranium ibericum, Geranium macrorrhizum, Geranium maculatum, Geranium maderense, Geranium* × *magnificum, Geranium phaeum, Geranium platypetalum, Geranium pretense, Geranium psilostemon, Geranium renardii, Geranium sanguineum, Geranium subcaulescens, Geranium sylvaticum;*
- *Pelargonium,* insbesondere *Pelargonium crispum, Pelargonium grandiflorum, Pelargonium peltatum, Pelargonium graveolens, Pelargonium denticulatum, Pelargonium x hortorum,, Pelargonium x asperum;*
- *Nicotiana,* insbesondere *Nicotiana tabacum, Nicotiana sylvestris, Nicotiana x sanderrae;*
- *Bouvardia,* insbesondere *Bouvardia longiflora;*
- *Vanilla,* insbesondere *Vanilla planifolia;*
- *Ipomoea,* insbesondere *Ipomoea batatas;*
- *Echinacea,* insbesondere *Echinacea purpurea, Echinacea angustifolia, Echinacea pallida;*
- *Schisandra,* insbesondere *Schisandra chinensis, Schisandra glabra, Schisandra rubriflora;*
- *Rhodiola,* insbesondere *Rhodiola rosea;*
- *Leucanthemum,* insbesondere *Leucanthemum maximum, Leucanthemum paludosum, Leucanthemum x superbum, Leucanthemum vulgare, Leucanthemum adustum, Leucanthemum graminifolium, Leucanthemum integrifolium, Leucanthemum lacustre, Leucanthemum monspeliense, Leucanthemum pallens, Leucanthemum praecox, Leucanthemum subglaucum, Leucanthemum sylvaticum, Leucanthemum waldsteinii;*
- *Strelitzia,* insbesondere *Strelitzia reginae,*
einschließlich interspezifischer Hybriden innerhalb der Gattungen und deren Nachkommen.

8. Verfahren nach einem der vorherigen Ansprüche, wobei Schritt (f) ferner die Auswahl einer reifen transformierten Mutterpflanze umfaßt, die nicht weniger als 80%, vorzugsweise nicht weniger als 90%, noch bevorzugter nicht weniger als 100% der Verzweigung im Vergleich zu der Wildtyp-Pflanze von Schritt (a) aufweist, aus der in Schritt (g) ein transformierter Steckling hergestellt wird.

9. Verfahren nach einem der vorherigen Ansprüche, wobei die erhaltene Pflanze nicht weniger als 90% der Anzahl der Blüten im Vergleich zu der Wildtyp-Pflanze von Schritt (a) aufweist.

10. Verfahren nach einem der vorherigen Ansprüche, bei dem die erhaltene Pflanze nicht weniger als 100% der Anzahl der Blüten im Vergleich zu einer nicht transformierten Kontrollpflanze aufweist.

11. Verfahren nach einem der vorherigen Ansprüche, bei dem die erhaltene Pflanze keine um mehr als 4 Tage, vorzugsweise um mehr als 2 Tage, verzögerte Blütezeit im Vergleich zu der Wildtyp-Pflanze von Schritt (a) aufweist.

12. Pflanze, erhalten durch das Verfahren nach einem der vorhergehenden Ansprüche, umfassend ein oder mehrere Gene, die aus dem Ri-Plasmid von *Agrobacterium rhizogenes* stammen.

## Revendications

1. Procédé pour obtenir une plante ayant une hauteur de 50 à 75 % de la hauteur d'une plante sauvage correspondante, et pas moins de 80 % du nombre de ses fleurs, comprenant les étapes suivantes
(a) transformation du tissu de la plante de type sauvage avec un ou plusieurs gènes provenant du plasmide Ri d'*Agrobacterium rhizogenes,*
(b) en permettant au tissu transformé de développer des racines,
(c) la sélection d'une racine supposée transformée ayant un phénotype de racine poilue;
(d) faire pousser la racine sélectionnée sur un milieu de régénération;
(e) permettre à une plantule à racine transformée de se développer à partir de ladite racine;
(f) faire pousser ladite plantule enracinée transformée en une plante mère transformée mature ayant une hauteur inférieure à 40 % de celle de la plante sauvage correspondante;
(g) préparer une bouture transformée à partir de la plante mère mature transformée,
(h) permettre à une plantule transformée enracinée de se développer à partir de la bouture transformée,
(i) en traitant la plantule transformée enracinée avec de la gibbérelline, ce qui permet à la plantule de se développer en une plante mature ayant une hauteur comprise entre la hauteur de la plante mère transformée de l'étape (f) et la hauteur de la plante de type sauvage de l'étape (a).

2. Procédé selon la revendication 1, dans lequel l'étape (a) comprend:
- à co-cultiver *A*. *rhizogenes* avec une plante ou une partie de plante, à permettre à *A*. *rhizogenes* de délivrer ledit ou lesdits gènes du plasmide Ri dans ladite plante ou partie de plante, et/ou
- l'infection par *Agrobacterium rhizogenes,* de préférence de type sauvage.

3. Procédé selon la revendication 1 ou 2, dans laquelle le ou les gènes provenant du plasmide Ri d'*Agrobacterium rhizogenes* comprennent un ou plusieurs gènes *rol.*

4. Procédé selon l'une des revendications précédentes, dans laquelle la plante mère mature transformée de l'étape (f) a une hauteur inférieure à 30%, de préférence inférieure à 25% de la hauteur de la plante de type sauvage de l'étape (a).

5. Procédé selon l'une des revendications précédentes, l'étape (f) comprenant en outre :
- le traitement de la plantule racinée transformée avec de la gibbérelline avant que la plantule racinée transformée n'atteigne la maturité, et/ou
- en testant la présence d'un ou plusieurs gènes provenant du plasmide Ri *d'Agrobacterium rhizogenes* dans ladite plante mère transformée mature.

6. Procédé selon l'une des revendications précédentes, dans laquelle l'étape (i) comprend:
- un traitement répété avec de la gibbérelline avant que la plantule transformée n'atteigne la maturité, et/ou
- permettre à la plantule de se développer en une plante mature ayant une hauteur allant jusqu'à 70%, de préférence jusqu'à 65% de la hauteur de la plante de type sauvage de l'étape (a).

7. Procédé selon l'une des revendications précédentes, dans laquelle la plante de type sauvage de l'étape (a) appartient à l'un des genres suivants :
- *Kalanchoe,* en particulier *K*. *blossfeldiana, K. laciniata, K. pinnata, K. marmorata, K. gastonis-bonnieri, K. dixoniana, K. humilis, K. ambolensis, K. aromatica, K. campanulata, K. citrina, K. coccinea, K. crundallii, K. daigremontiana, K. decumbens, K. faustii, K. fedtschenkoi, K. figueredoi, K. flammea, K. glaucescens, K. gracilipes, K. grandiflora, K. guignardii, K. jongmansii, K. laciniata, K.latisepela, K. laxiflora, K. lobata, K. longiflora, K. manginii, K. nyikae, K. obtuse, K. paniculata, K. porphyrocalyx, K. prittwitzii, K. pubescens, K. pumila, K. rauhii, K. rotundifolia, K. scapigera, K. schumacherii, K. spathulata, K. streptantha, K. synsepala, K. tomentosa, K. thyrsiflora, K. tubiflora, K. uniflora;*
- *Chrysantemum,* en particulier *Chrysantemum morifolium, Chrysantemum x morifolium (syn. C. x grandiflorum p.ex. Dendranthema hybrids,* ou des hybrides entre *Chrysantemum morifolium* et d'autres espèces de *Chrysantemum,* comme *Chrysanthemum indicum;*
- *Aster,* en particulier *Aster novi-belgii, Aster dumosus;*
- *Rosa,* en particulier *Rosa hybrida, Rosa canina, Rosa spinosissima, Rosa damascena "trigintipetala', Rosa centifolia;*
- *Solanum,* en particulier *Solanum lycopersicum, Solanum tuberosum, Solanum nicotiana;*
- *Euphorbia,* en particulier *Euphorbia pulcherrima, Euphorbia milii;*
- *Phaelanopsis,* en particulier *Phalaenopsis amabilis, Phalaenopsis amboinensis, Phalaenopsis aphrodite, Phalaenopsis appendiculata;*
- *Ocimum,* en particulier *Ocimum basilicum;*
- *Capsicum,* en particulier *Capsicum annuum, Capsicum baccatum, Capsicum chinense, Capsicum frutescens, Capsicum pubescens;*
- *Mentha,* en particulier *Mentha arvensis, Mentha requienii, Mentha spicata, Mentha longifolia, Mentha pulegium, Mentha suaveolens, Mentha aquatic, Mentha arvensis x spicata, Mentha aquatica x arvensis, Mentha x piperita;*
- *Hibiscus,* en particulier *Hibiscus rosa-sinensis, Hibiscus schizopetalus,* - *Hibiscus sabdariffa, Hibiscus syriacus, Hibiscus trionum, Hibiscus cannabinus;*
- *Mandevilla*/*Dipladenia,* en particulier *Mandevilla* ×*amabilis, Mandevilla sanderi, Mandevilla splendens;*
- *Eustoma,* en particulier *Eustoma russellianum, Eustoma exaltatum;*
- *Lavendula,* en particulier *Lavandula angustifolia, Lavandula; latifolia,Lavandula lanata,Lavandula dentate, Lavandula stoechas, Lavandula pedunculata, Lavandula viridis;*
- *Lillium,* en particulier *Lilium bolanderi, Lilium* × *burbankii, Lilium canadense, Lilium columbianum, Lilium grayi, Lilium humboldtii, Lilium kelleyanum, Lilium kelloggii, Lilium maritimum, Lilium michauxii, Lilium michiganense, Lilium occidentale, Lilium* × *pardaboldtii, Lilium pardalinum, Lilium parryi, Lilium parvum, Lilium philadelphicum, Lilium pitkinense, Lilium superbum, Lilium ollmeri, Lilium washingtonianum, Lilium wigginsii;*
- *Clematis,* en particulier *Clematis addisonii, Clematis albicoma, Clematis alpine, Clematis aristata, Clematis armandii, Clematis baldwinii,Clematis bigelovii, Clematis brachiate, Clematis campaniflora, Clematis catesbyana, Clematis chinensis, Clematis chrysocoma, Clematis cirrhosa, Clematis coactilis, Clematis Columbiana, Clematis crispa, Clematis dioica, Clematis drummondii, Clematis durandii, Clematis ispahanica, Clematis fawcettii, Clematis flammula, Clematis florida, Clematis fremontii, Clematis glaucophylla, Clematis glycinoides, Clematis henryi Clematis hirsutissima, Clematis integrifolia, Clematis* × *jackmanii, Clematis lanuginose, Clematis lasiantha, Clematis leptophylla, Clematis ligusticifolia, Clematis macropetala, Clematis marmoraria, Clematis microphylla, Clematis montana, Clematis morefieldii, Clematis napaulensis, Clematis occidentalis, Clematis ochroleuca, Clematis orientalis, Clematis palmeri, Clematis, Clematis patens, Clematis pauciflora, Clematis pickeringii, Clematis pitcher, Clematis recta, Clematis reticulate, Clematis rhodocarpa, Clematis smilacifolia, Clematis socialis, Clematis stans, Clematis tangutica, Clematis terniflora, Clematis ternifolia, Clematis texensis, Clematis versicolor, Clematis viorna, Clematis virginiana, Clematis vitalba, Clematis viticaulis, Clematis viticella;*
- *Geranium,* en particulier *Geranium cinereum, Geranium clarkei, Geranium dalmaticum, Geranium endressii, Geranium fremontii, Geranium himalayense, Geranium ibericum, Geranium macrorrhizum, Geranium maculatum, Geranium maderense, Geranium* × *magnificum, Geranium phaeum, Geranium platypetalum, Geranium pretense, Geranium psilostemon, Geranium renardii, Geranium sanguineum, Geranium subcaulescens, Geranium sylvaticum;*
- *Pelargonium,* en particulier *Pelargonium crispum, Pelargonium grandiflorum, Pelargonium peltatum, Pelargonium graveolens, Pelargonium denticulatum, Pelargonium x hortorum,, Pelargonium x asperum;*
- *Nicotiana,* en particulier *Nicotiana tabacum, Nicotiana sylvestris, Nicotiana x sanderrae;*
- *Bouvardia,* en particulier *Bouvardia longiflora;*
- *Vanilla,* en particulier *Vanilla planifolia;*
- *Ipomoea,* en particulier *Ipomoea batatas;*
- *Echinacea,* en particulier *Echinacea purpurea, Echinacea angustifolia, Echinacea pallida;*
- *Schisandra,* en particulier *Schisandra chinensis, Schisandra glabra, Schisandra rubriflora;*
- *Rhodiola,* en particulier *Rhodiola rosea;*
- *Leucanthemum,* en particulier *Leucanthemum maximum, Leucanthemum paludosum, Leucanthemum x superbum, Leucanthemum vulgare, Leucanthemum adustum, Leucanthemum graminifolium, Leucanthemum integrifolium, Leucanthemum lacustre, Leucanthemum monspeliense, Leucanthemum pallens, Leucanthemum praecox, Leucanthemum subglaucum, Leucanthemum sylvaticum, Leucanthemum waldsteinii;*
- *Strelitzia,* en particulier *Strelitzia reginae,*
y compris les hybrides interspécifiques au sein des genres, et leur descendance.

8. Procédé selon l'une des revendications précédentes, l'étape (f) comprenant en outre la sélection d'une plante mère mature transformée, ayant au moins 80%, de préférence au moins 90%, de préférence au moins 100% de la ramification par rapport à la plante de type sauvage de l'étape (a) à partir de laquelle une bouture transformée est préparée à l'étape (g).

9. Procédé selon l'une des revendications précédentes, dans lequel la plante obtenue ne présente pas moins de 90% du nombre de fleurs par rapport à la plante sauvage de l'étape (a).

10. Procédé selon l'une des revendications précédentes, dans laquelle la plante obtenue ne présente pas moins de 100% du nombre de fleurs par rapport à une plante témoin non transformée.

11. Procédé selon l'une des revendications précédentes, dans laquelle la plante obtenue n'a pas un temps de floraison retardé de plus de 4 jours, de préférence de plus de 2 jours, par rapport à la plante sauvage de l'étape (a).

12. Plante obtenue par la méthode selon l'une des revendications précédentes, comprenant un ou plusieurs gènes provenant du plasmide Ri *d'Agrobacterium rhizogenes.*


### SEQUENCE LISTING

<110> Knud Jepsen a/s
<120> Method for obtaining plants with intermediate height
<130> 60850NL
<150> NL2012284
   <151> 2014-02-18
<160> 18
<170> PatentIn version 3.5
<210> 1
   <211> 19471
   <212> DNA
   <213> Agrobacterium rhizogenes
<400> 1
   tggcaggata tattgtgatg taaacagatt agatatggac atgcgaagtc gttttaacgc     60
   atgctttatc gaatataaaa tgtagatggg ctaatgtggt tttacgtcat gtgaataaaa    120
   gttcagcatt cgtttaataa tatttcaata tcggtgtcta gagacccgtg gatttgtata    180
   gtcagcacca tgatatgaat ctataaaata ttgtatctcc aattgcaatt caatcgatat    240
   aagaaattaa tacaagccgt tcatatagta aggttgccaa tggcattcaa taacgaccgt    300
   acagttgccg ctatattaat ctacgtgcca tttcttaaat aaagataggc gaatgactat    360
   cgaaaataaa acaattatta atgagtgaaa acgtattgca caaataaaga ttcattatgg    420
   ttggctcaaa ttttggctct ggtgctcgat gacgtcgaga tgaggacagt agtgatcaac    480
   ttggcggtcg ataccttggt tacgccactc ccagagtgcc atgtcgtcct ccgagcggtc    540
   tgagataacc cagtcggcaa ttgctgctgc attgccgggc gttccccaac cacgacgaat    600
   atgctttcgt tcatctaact cgcgtcgcac tgccctccca gtcatgaagt caaagccaaa    660
   ttctaccctc tctccatttc ccagctcagt cgagaaatcg taacacctcg tggcagctga    720
   cagtttcaga aaggggcgta tccctcgaac tccagggtcc tctttcacat agttagcaag    780
   gcgtactgct gcataatctg cgttgaaggc tctgatgact acaggatcct cggacaagcc    840
   caattgatca gggcgaaccc tcgcgctcat aatatgaatt gcgacgaccc ttgcttcctg    900
   tcggagcatc gaatcaatcc aagccttccc tgcggcatag aggtcatcga ctgcgatgtc    960
   atcaagatcg agtagctttg ccaacctagg aagttcttga ggaaaaatca ccggcatgac   1020
   agcaaccgtc tctcgccagt cagttgccgg actggcttcc ctaacgccat ccacgaatgc   1080
   ctcaccgctt gcgtatttga atgtgtaaaa gagaaggacc actctttggc ggtacttcgg   1140
   acgccggctt agccacgcgg caataatgtg ggcctcaaac tcacgaccat ccaaaaatat   1200
   agtcgcgcct ggattgacct cgctggcctt gtcgagaaga ggttccaaaa agggaacggt   1260
   gtctttcgta atagtactta aatctgtgag ttcgccatgc gaaacctctc gaacgattat 1320
   cggcgtatcc ctgacatcag ctgaatgaaa ttctcggacg agtttgtcgg gcaaagtgga 1380
   gacccgccac gtgttgaagt cgtgggaaac gatgggcaca tcgtcgccgg tgagtgcggc 1440
   atcgagctca gagaggttcc gcctgccaac ctcaccgaga gcagctaaca acgaagtttc 1500
   ggtgcattcc tgtatccctt tacccagatt atacatgccc cggtgttcga taacttgaag 1560
   aggcagtggc tcctcaagat gttcaaggag gtggggtaca gagtgccggg cgaggacctc 1620
   atccaccgtg acaccaaccg ggagatccca ttcgagtttc cactggggcc agcatgtgcc 1680
   cgcgacggcg aaaggtttgc gctggcaaag aacccggctg ctgcaggtgg acctatcctt 1740
   acccatggca atggggtttt gctaaaaagt caggcacttt actgggcaat tgatagggtg 1800
   ggattgcgtt attaactgtt ctccagcggg aatctttatc tttattgaaa tgctaaagca 1860
   cttagataaa atacagctgt accgcaatat aaaatagtag gataatgtaa tatgtgtatc 1920
   gagaatacga caagctaata taatctagcg tcaaattgca ataatttaaa tcaaaactac 1980
   tgatgaaata ataaaagatg gtcaattttt attggtagga gttgtcgaaa gattcgacgg 2040
   acggccatta caatacatag gtgcaagaag taaaacagga agggaaacgg aaaacagtgc 2100
   tataaaaaag cgacagatcg cggcgatcac tgactgcgat cgggaagaag ctcgccaagt 2160
   tcaccgagaa tagcagagag cgcatcctca tcgggtacta cgaacacatt cgtcccagag 2220
   ggctttgttt cagctgcgcc aacccagaaa gcaaggccat tttccaagtt gccgatggcg 2280
   gtcagcatgt tttgattgtt gctgccgttt ccacaagcga tgtgaaggcc gatcccgtga 2340
   gagaggccct tgacgaaggt gaaatagcct ttggattttc caactgtttc aacgggcact 2400
   agatattgac cctctggcgc ggcaaccacc ttgaatttgc gagatgactg gttgccgatg 2460
   agcgaagaaa gcatttctcc ggcttctttg taagatttgt gagattccca catttgacag 2520
   ccgtagaaat gccccatcgg aatgttgcgg attcccggga tgccaccaaa tttgttctcc 2580
   atagccgcgt gaacggcttg ccagttgggc agggagaaag aatcgaagcg atcatctttg 2640
   tagatcgtga ccattccatc atttccctgg aatccgatat tttcaatggc gctgaaaact 2700
   gaccttgcga tttcttcgca ttcccgtgcg gatgtgagca attgataatg gcccttgcag 2760
   gcgatcctgg tcaaattggc gatgatgttg atggcaggat taatatccca acactggtga 2820
   tttcgatctt gcttaaaggt ggtaccatcg ccgtcgaagg cgagcagggc ccggagagat 2880
   gaatcggcaa gactgcgtcg gacccgctcc gcggcgtcgg gaatgaggct gataagagac 2940
   atatccaaag gtgtttgtgg gtaacgggct gctcaatgaa gccttaaatg caacgcaaca 3000
   tatgtaagga tgagttgact tattggagag agaaatagga atgagctggc cagccattat 3060
   caacgtgggg ccatgctgac aatgtttacg tgaaaggctc aactacctcg aagcagacct 3120
   ctatattcgt tgactttatt actgaacaag aagttgcttg ccactcattt tcttaaatct 3180
   tgccctttct gcgcctcgct atcatgcccg ccaacgacgc gacatgcgct gccgcgattg 3240
   ccttccccga gggcaactgg aaggaagaac ttgatgcgct ccgcaccttg tgtgaccccg 3300
   tcgaggtggt taaggtcgca gtcggcagag gtcttagcgg catatgtaat gttgttgcag 3360
   caatgaatcc cacaaaggtg aggggcctcg gcgatgtcat cgggcagatg ccggctctta 3420
   atcaccgtat tgctgccgcc gccggcgaaa ctccggtgcg agaccttgga ataggttacc 3480
   agtgcgcaat ctgccacccc gacatagcca gtgcgatgtt agccacttct gaggggatca 3540
   gccacgttct ccgtgaaagg attgagaaag aagttgaccg ggacattgga gaaggcgcca 3600
   ccgtctgcat tttcgttcag ccgagaatga gctccaaggg ctctccagtt tctgtccatt 3660
   tcaccctcca gtttgcgaga tctggaactc ttgtcgatgc cagaatgatg gagagttaca 3720
   atttcatgaa aggcaatggc acagtgaccg caccggattt gaaaagtcat tggaagaagc 3780
   acggtattga caggccaggc ccacgtccgc ccacgtccaa gtttgaactc ctcttcgccg 3840
   ctgtccccga caacagtaaa cttgccgcca ccgattttac ccatctcggc cctgtcgagc 3900
   gtgataagga actactcggc agcacggtat tcgggattgc cgctaagaaa cctggtacga 3960
   tcgtttatcc gtgcgaaaag gttctctgtt tggaggtcga cgtacacgcg catcgcgccc 4020
   tagaagtact tcaccgcctt ggggaacagg cttatagcaa tggccgtggc actagcttcg 4080
   gtcttcacac cggtccgtcc tcttgcctta atctttccgc cgccgcgctc gctacatttt 4140
   tcaaacgctc ggatctctgt tcccttccat tgagtgatgc ttttgtcctt ttctgcgacc 4200
   cgccaccgcc tacagcgcca agaaagatgg ccttccgatc actgccttct cccccacgag 4260
   caccaatcag ttcgaactcg tagagcctca ggtcgtcaag gcatatgttc tcggactttt 4320
   cgacgcgccg acgatggtta cgccccgcga caaaacgcga gccagcttct gcagccaata 4380
   tgtacgtttc cgtgaaccgc atccctgtga agagttcaat gaaattggag ttttgatcct 4440
   cgatgctgct gctaaaatgc tcgaacgtta tgcaaaattt ctagaagatg gtggaagaga 4500
   tgatgatgaa atggcgaaca taatagatgt atttgggttt tgtcttaact agtggattga 4560
   ttgaaacaaa ggagtccgag ttgggattcc ctttcggtct tcgtcgtgca acgatatcgt 4620
   atgcgtacag gtatcacatt taacgttgct gcggcggacc gagcccgctt ggaagcgatt 4680
   gttgcagctc caacttctgc tcagaagcac gtgtggcgag cgaagatcat cttgatgagc 4740
   agtgatggct cgggaacggt cgcgatcatg gaggcaaccg gtaaatccaa aacctgtgtc 4800
   tggcgctggc aggagcgctt catgactgag ggcgtcgatg gccttttgca cgacaagagc 4860
   agaccgcccg gcattgcgcc gcttgatggc gaactcgttg agcgtgtcgt cgcactgacg 4920
   cttgagacgc ctcaacagga agcaacgcac tggactgttc gtgcgatggc caaggccgtt 4980
   gggattgcag cctcttcggt tgtgaagatc tggcacgagc atggtcttgc gccgcatcgc 5040
   tggcgctctt tcaaactgtc gaacgacaag gcctttgccg agaagcttca cgacgtcgtt 5100
   ggcctctacg tctcgccacc ggcccatgcc attgtcctgt ccgtcgatga gaagagccag 5160
   atccaggcac tcgatcggac gcaaccggga ctccccttga agaaagggcg cgccggcaca 5220
   atgacccacg attacaagcg ccacggcacc accaccctat ttgccgccct caacatcctc 5280
   gacggctcgg tgatcggccg aaacatgcag cgtcaccggc atcaggagtt catccgtttt 5340
   ctcaacgcca tcgaggcgga actgccaaag gacaaggccg tccacgtcat tctcgacaat 5400
   tacgcgaccc ataagcagcc gaaggtccgc gcctggctgg caaggcatcc gcgctggacc 5460
   ttccacttcg tcccaacatc atgttcatgg ctgaacgccg tcgagggatt cttcgctaaa 5520
   ttgacacgtc gacgtctgaa gcacggtgtc tttcattccg tcgttgacct ccaggccacc 5580
   atcaaccgct tcgtcagaga gcataatcag gaaccaaagc cgttcatctg gagagcagat 5640
   ccagacgaga tcattgcagc cgtcaaacgt gggcaccaag cgttggaatc aatccactag 5700
   cgtatgaaca gtaataagaa aatcccgatt gtgaatagtc ccaatttcaa atgtgtccgt 5760
   gtgtaatttg cgtgtcttca gttgaatttc ctttaataat atcaaatatt caattgtgaa 5820
   aagttgtatt ggttcaggtt caagctttcc gaatttgttg aattttattc cctgttttca 5880
   atttgttgac ttgtttggga gacacctttt ttgtgtttcg tgaacatgtc accccttcgg 5940
   tatacattag cctacaaagt aaataacgtt gataaatgtc actcatgttg taataaaatt 6000
   gagcttatta tgtataacca gaccctgtgt taatctaatt acaaagaaat tcatcattct 6060
   cccaagcaat cctgagtagc tgcgtgatgg atcttccata tcagcgccca cgtttcaccc 6120
   cgtttgccgt cacccatcca cgtagtggag tcaacctgaa ccgtgcaatt tctcaggcct 6180
   ttgtctgcta tgatcagttc tgcgaacggc tcttgcgata tcagcaaagc tggacggatt 6240
   gggtgttcga ccacggattt gcagaagcca ttgaagacgt ggcgctggtg ttccaggttg 6300
   caccttgcct tcatggcccc cgaataggcg cgctcgaagt gttgatacct cgtcgcaccc 6360
   aggtcttcat ttatatgtcg aacaaccaat tgcagcgctt tgttgcacac cagtgcattg 6420
   ctcaacttgg cgacgccgtg cttgcttgca tgatcccgcc ctacgcgagt gacctctcgc 6480
   tgcaggaaat ggctcgggcg cacaacagat tttgcccagg cagttacacg aggtccgcag 6540
   acgtacagtg ctttatcgcc atccaactca gcagccgatt cgttgaggag ggcacatgta 6600
   acgtgcacgg gcgaaatggc ttaaaaagaa cctgccgctt ctttcgtcgc cctgctgagt 6660
   tcttcagccg ttatgacatc gttgccattg ggccggtgct cttccatgat gaactggatt 6720
   gcccagcaaa ctgcaatgag cctctttcct gctttgacct gcggtacgac tatcaggttt 6780
   tcctccagga gtgcgatgcc catgatggtg tggggcatta tccggaaggc gcaccactac 6840
   ctagtgttgc catcgtagga ggcgggctgt ctggccttgt tgctgccaca gaactacttg 6900
   gcgctggcgt caaggaaatc actcttttcg ataccgttga tgagatccgt agttttgggg 6960
   catcgccgat gccaaacggc gacgctcacc aggccttgac gtcgttcggt gtcatgcctt 7020
   tctccgccaa ccaactttgc ctgtcatact atctggataa gtttagaatt ccgtccagcc 7080
   ttcgttttcc ttgtgccggc aacgaccaca cagcactata tttccgccag aaacgctacg 7140
   catggcacgc ggggcaagct ccgccgggga tatttcagcg ggtacatgtc ggatggaaga 7200
   cactactcta ccaagggtgt gaacggaatg gcaggagact gatggctccg atggatatct 7260
   ctttcatgtt gaaagagcgt cgtcgtgatg aagcctcaga agcacggcag ctttggctcc 7320
   gagagttcgg aaaattcact ttccatgccg ttttggtcga gatcttcagc tgtggtaatt 7380
   cgagtcctgg tggcaaggca tggcaaacac cccatgattt cgaggctttc gggatactga 7440
   ggttgggata cggccgagtt tcgtcctatt acaacgtgtt gttttcaacg atcctggact 7500
   ggattatcaa tggctacgag gaggaccagc atctttctat tggtggggtt caacttttgc 7560
   aggctctgat gcgcattgaa atattccaga aaagccatgc gaaagcacga ctctgttttg 7620
   atcccgtgcg tggaatagcc aaggagggcg ggagattgaa ggtatgcttg aaacacggtc 7680
   attcgcgtgt ttttgaccag gtcatcattg gcggcagtgc tgaggccgct acagttgata 7740
   acagactggc cggggatgag acttccttca gctacaatat cgaacccgcc gtcggaaact 7800
   cgtctgccgc tgtcaattca gcactcttca tggtcacgaa gcaaaagttt tgggttaact 7860
   ccggcatccc agcagtgata tggaccgatg ggcttgtccg tgagctgtgt tgcattgaca 7920
   tcgaatcgcc agctggagag ggccttgtcg tttttcacta tgctttggat gactatctat 7980
   cccggccgat cgagcatcat gacaagaagg gacggtgctt ggaattggtc agggagcttg 8040
   ctgctgcctt tcctgaactg gcttgtcacc tggtcccagt caacgaagac tacgaacgat 8100
   atgtcttcga cgaccaccta acggatggtt ttaagggagc tttgtggagg gaaaattctc 8160
   tggaaaaagg tcagtatatc caggatctgc ctgggaataa ttttcctatt ggggatcacg 8220
   ggggagccta tctgattgac cgtgacgact gcgtcaccgg agcctcgttc gaggagcagg 8280
   tgaaggcggg catcaaagcg gcctgcgccg tcatccgcag caccggcggg acgctctctt 8340
   cactccaacc ggtggactgg aataaaaaat agaaatttcc tgattaagtt atagtcaatg 8400
   tactattgcg tgttaatccc gtaggtatgc aagctgcacc ggcagcatca taatttgatg 8460
   ttccatcaat aaattaaggt gcccgttcat tgtgtattac attatgtatg tttatcaaaa 8520
   atataatcga agtccatttt aagtctgata ttaattggaa ttccaaacga ttccttgatg 8580
   cctatcttcg ctatgattgt atggtaataa agtctccaca tctcccgaaa aatgctttcg 8640
   tgatttactt gtctctcacg tgctttcgca tcttgacagc caaaagtggg caacttgaga 8700
   agagtattaa ctggccacgc aactcgagat attcccacta accccaatga cgtcattgca 8760
   ctcgtcacgg gtagcagccc cacttgcctt tgccacttta ttaattcttt ggcccactgg 8820
   ccattaattg gcacctacat atattagtgg agaagataaa gtgtcactat cgtttcctgt 8880
   tcaattttga attttgcaag gatttcatgt tgtcaactac acagcttgaa aggaaatccg 8940
   caatcaacgg agaaacgtca acatctcgac aaaaaaagaa tgcttcatca ttgcgtagac 9000
   tgcatattga ccgctccttt cggcgctggg cctgctttta ctgttgccta gcgttcggac 9060
   agccaccaga gaatgggcta tatagatcct ttcatcaaac caaaacatta ctaagatcat 9120
   gctgtaacgc ttcaatacgg tgagtgtggt tgtaggttca attattacta tttttgaagc 9180
   tgtgtatttc cctttttcta atatgcacct atttcatgtt tcagaatgga attagccgga 9240
   ctaaacgtcg ccggcatggc ccagaccttc ggagtattat cgctcgtctg ttctaagctt 9300
   gttaggcgtg caaaggccaa gaggaaggcc aaacgggtat ccccgggcga acgcgaccat 9360
   cttgctgagc cagccaatct gagcaccact cctttggcca tgacttccca agcccgaccg 9420
   ggacgttcaa cgacccgcga gttgctgcga agggaccctt tgtcgccgga cgtgaaaatt 9480
   cagacctacg ggattaatac gcatttcgaa acaaacctac gggattaata cgcacgtggc 9540
   tggcggtctt cgattcattt ccacgccgga gatgatatcg aatatgttct gttaagttaa 9600
   aataagctgc gagccatggc gcgattgtcc tgttttatta atatagtact ttaacgtctc 9660
   tttagagcgt ttgtgtaatg tcgtgaaaat gttttatgtc aaatgtactg ttgaactata 9720
   atattataag tccaggtgtg tcgttgttgt tgatactgca atatatgtgt agtagattag 9780
   atagtcatat gagcatgtgc tgtttttggc aaaattcagc agcaggatca acacagaaga 9840
   aaatatttag tacaagaaaa taggtcaaca cattacaacg tacgctacaa ctcccaaggt 9900
   tctgtgtcac agactgcggg agggtacata gaacttatga caaactcata gataaaggtt 9960
   gcctgcaggg ggagttcaag tcggctttag gcttctttct tcaggtttac tgcagcaggc 10020
   ttcatgacgc cctcctcgcc ttcctgatca ggccccgaga gtcgcagggt taggtctggc 10080
   tccggtgagg aggcggccgg acgtgatatc ccgagggcat ttttggtgaa ttgtgtggtg 10140
   ccgcaagcta caacatcata ggggcggttt tcagtccctc gccgcagaaa gaaggtgcaa 10200
   gctacctctc tcccgtaaac gttggtcact tttaactcca gcaagtgaat gaacaaggaa 10260
   cttgcgaaaa tggcgatgaa gcattctaaa tcaggttcct ccgtgcggct gtgcggccaa 10320
   gcaaggttgt gaacacggag catctcctgg agggcgagct cgctccgata tggttgaatc 10380
   gttgtcgcca gcacggcctc cattccaaat gtaatggatt gttccttcag cactttctgc 10440
   atcttctcgc gagaaagata gacaaataca tgttggtcgt tttctcgagc cagatccggc 10500
   tgactaacaa acataggagg atgatagcag actttgttct tcaagagctc agctagttgt 10560
   ttaagtatat atatcggtgg agagttttcc ttcaaatcta gcactgcaag agcccatcgt 10620
   ttctggaaat gcaggagggg tttgctatag tcacggctat agattgcaaa agcaaatcgg 10680
   atcccctcga ataggtttat ctggctccat gctggagtga gatctactgg ttgaaatcgt 10740
   ggaaggaata gcaatttggg atccattgtg atgtgagttg gatagttacg aaaaaggcaa 10800
   gtgccagggc catttaaaat acggcgtcgg aaactggcgc caatcagaca cagtctctgg 10860
   tcgggaaagc cagaggtagt ttggcaacaa tcacatcaag atcgatgcgc aagacacggg 10920
   aggccttaaa atctggatca agcgaaaata ctgcatgcgt gatcgttcat gggttcatag 10980
   tactgggttt gctttttctt gtcgtgttgt ttggccttag cgaaaggatg tcaaaaaagg 11040
   atgcccataa ttgggaggag tggggtaaag cttaaagttg gcccgctatt ggatttcgcg 11100
   aaagcggcat tggcaaacgt gaagattgct gcattcaaga tactttttct attttctggt 11160
   taagatgtaa agtattgcca caatcatatt aattactaac attgtatatg taatatagtg 11220
   cggaaattat ctatgccaaa atgatgtatt aataatagca ataataatat gtgttaatct 11280
   ttttcaatcg ggaatacgtt taagcgatta tcgtgttgaa taaattattc caaaaggaaa 11340
   tacatggttt tggagaacct gctatagata tatgccaaat ttacactagt ttagtgggtg 11400
   caaaactatt atctctgttt ctgagtttaa taaaaaataa ataagcaggg cgaatagcag 11460
   ttagcctaag aaggaatggt ggccatgtac gtgcttttaa gagaccctat aataaattgc 11520
   cagctgtgtt gctttggtgc cgacaggcct aacgtggggt ttagcttgac aaagtagcgc 11580
   ctttccgcag cataaataaa ggtaggcggg tgcgtcccat tattaaagga aaaagcaaaa 11640
   gctgagattc catagaccac aaaccaccat tattggagga cagaacctat tccctcacgt 11700
   gggtcgctag ctttaaacct aataagtaaa aacaattaaa agcaggcagg tgtcccttct 11760
   atattcgcac aacgaggcga cgtggagcat cgacagccgc atccattaat taataaattt 11820
   gtggacctat acctaactca aatattttta ttatttgctc caatacgcta agagctctgg 11880
   attataaata gtttggatgc ttcgagttat gggtacaagc aacctgtttc ctactttgtt 11940
   aacatggctg aagacgacct gtgttctctc tttttcaagc tcaaagtgga ggatgtgaca 12000
   agcagcgatg agctagctag acacatgaag aacgcctcaa atgagcgtaa acccttgatc 12060
   gagccgggtg agaatcaatc gatggatatt gacgaagaag gagggtcggt gggccacggg 12120
   ctgctgtacc tctacgtcga ctgcccgacg atgatgctct gcttctatgg agggtccttg 12180
   ccttacaatt ggatgcaagg cgcactcctc accaaccttc ccccgtacca gcatgatgtg 12240
   actctcgatg aggtcaatag agggctcagg caagcatcag gttttttcgg ttacgcggat 12300
   cctatgcgga gcgcctactt cgctgcattt tctttccctg ggcgtgtcat caagctgaat 12360
   gagcagatgg agctaacttc gacaaaggga aagtgtctga cattcgacct ctatgccagc 12420
   acccagctta ggttcgaacc tggtgagttg gtgaggcatg gcgagtgcaa gtttgcaatc 12480
   ggctaatggt tagtcgatgg gctgacgagt ttgatgtcag gagaagctga gtgtgtcact 12540
   tgtttccctt taagaagtat taatgtaata aaaatcaaga tctggtttaa taactggata 12600
   cttgatttca tcgcgctttt tttgaataaa tgtttgttgt cttgacttta agatatcctt    12660
   tgaaatttgc gttattcgta tttcgctttt ggttatttcc aaaagacttt gctcagtaag    12720
   atcaaacgtt tgtatttctc cgggccacaa tatttgacct atatgcactg gcccacgcgc    12780
   cgcaatagat gaaaattgcc aaaattagct atcggtcttc tgaaaagaag ggccgacatg    12840
   ttttcataga ccatgcaaag tcatactacc tgaaactgat aaataacgac aaagaaagta    12900
   gcctatttaa aagtcgctat agcatgaatt caacacaagg aaaccaaaag tcggaaggaa    12960
   gactttaatc ccggattatt tggacatgat aggagctatg gggcaacgtg tcattttcat    13020
   gagtgttgaa tgattttctg tagcaaatag aaaacgtttt ttaaaacgat gtggccttgg    13080
   agtaatcagc ggaagaaatg gtcatgctca gataatttcc gttgctgacc tcgcaaccaa    13140
   cccctttaaa tacctctgct gcccatgcat tttgccaagt taacctaaag tggcagctga    13200
   atggctcgtt attgcagtgg tggctctcaa cggcttcatg tcgatgattt tcgttggatc    13260
   aaggagccca ctcgactgaa ggctcagctt attaatgtgg tggagaccta caaggctgca    13320
   caaacagaga cgttaaagta ctatatatca tctgcaactg agcgtgtggc tcatgtggag    13380
   gcagccgagg tcaacaatgc ggaaatggag ctgcatcctg ctgggttgaa gtaccctctg    13440
   tccttcgtct ttacctccct ggccgtggct acagcctgca aggagaacaa gcatctcttg    13500
   tgcgaggagc atttggaggg ggacttgata tcgtgcgtcg ttcctcccta tcagacaaat    13560
   gtctcactcg ctgctttaag ggagctccac aattccattt cgggaggagg gtaccaggaa    13620
   caagcagaca tggattattt tgtggcgatc atcccaaatg ataatttcga ctatcagagc    13680
   tgcgaaatcg acacacgaag ttgcggtaaa ggactttgca agatttatag tagggaactg    13740
   ggagggcagc ctctagctta tgacgccata ctggcaatcg gcaaggtgct gctgctggaa    13800
   tagatagtgg gccgctgatc cgagtttgat tttgtcgtat tatgttacgt gaacttttta    13860
   tcatgcatgt ttcgcttatg ctcccgagtg tcggccatgt tgttgtgtta aaataaaagg    13920
   ctgatgttaa gtcctattgt aaaatacctt tatagattaa atatatatag tataacttct    13980
   gtatgccgtc gatgagcggt tatatgattg taatctatac gttgttgcaa tcaatcgtat    14040
   tacagtgagc cgtgcttaat gaaataaaca tcatgttaaa tgtctattta ttcaatcaac    14100
   atgcgctgac aataatcaaa aggggaaacg taataacatt gcggtggata cagcgtttat    14160
   tgggaggtcc gcgggccgat acacttaaat aacatagaca gaatttgaga gagcacgcag    14220
   gttgtagcca agttgagcga cttgccggta gcacggaagc taagctcagg tgttacaaat    14280
   agacaggcgt cgaggcgacg agcacgacga ccttgccgga cattgcggtc gcagggggct     14340
   caaagcggtt ggcttgtaac ggaccttgtg tttcttgttg tagctttcat cgagcataac    14400
   cattgggacg gttgctgaac aacggtaacg cacttttttc acgggagcga ggtagaagaa     14460
   catatttccc cgtcggcagc cggcggtgag catgccaatt cctaagggat caatggactc    14520
   gtgcgaacgg tgagcatgcc gttctgaccg tcggtgccca atcagcaggc cactcccaac 14580
   atgttttcca agtccttaaa accagtcttt atagcattga tctcccagca atctttattg 14640
   aagtcgattt taatattcaa aagaagattt tagtggaaag ggaatataat cgcgtggccg 14700
   aagaagagcc ttcaaaaatc agaatccact aggataaaca ataatatctg aaaagcattg 14760
   aatttgggtt aggcacgaga ggctgacgcg gatgccactc gattgctagt ggaaggattc 14820
   ccttttttct agcgtatcga attcaccgtt tcactatatg ttttcctgat tggttgatct 14880
   gcgggaccac cattgactgc cactaatatc gaaagtgggt ctgcttcgat tatgatgctt 14940
   tgtgagaggt tctcttccca atgcatgcaa gctggcagat tcggatactc tcaatagaga 15000
   tcttatttcg cgtctcaaaa agttcccaga aatcaacaaa ggggagggca ggtcctttaa 15060
   atacgttgca gctgtccttt aaaatagaag agaatttaca gctggaggca cagaccacta 15120
   aactgcgaaa gtaagcatgg cagatgagtt ggagcgtcaa ttggaagcca tttctctcat 15180
   tacagtcctg ggtccggatg tgaaggctga gcttgaggcg gagctacgag actactgcga 15240
   agatctcgac ttctggaaaa gccacggttt accggtggcg gatctcgatc agactgtgac 15300
   tgtcgacaag cttctataca tgtatatgga tcgggcaaca gcagacctgt gtgtgaagaa 15360
   tcgctgcctc gtttgcaaca gtggcaattc agccgcaaaa gtaacctcgc ttccaccata 15420
   ccttgcaggc gtgacaagcg ccgaggccta tgagaaactc aactccattg ttgatgggag 15480
   tgtcgccccc caatctcgtg ggcctccctg ctattttgtg gcgttcctgc ccagcagctg 15540
   tttcgagaaa accagtgaga tatcggtgcg cacagtggac ggcgagtgtg gccccttcga 15600
   tgtctttacc cggcagcgtc agccacagga tcagagtgat atgtttttta aatatgaagg 15660
   agttgtatgt gctggaaaga gtgtatttat gtaagaatta tcttttatag cctgtgttac 15720
   gtttgaaccc ggtccgcgcg gtattgtttt caataaatgg tatgtgcgga ggatataatt 15780
   ggtctttcat tggtgtgatt tacgtgtaac gcggataata ataaagtaaa ttacaaaaga 15840
   gaaacgcata attttattcc agaatgattg cgagaaacga tgaaaataca tgaaaatgca 15900
   tattgtcgcc agggaaggat ggcgccgaaa taaacgaaac tgagccaata cagtgacttg 15960
   ccaagcgagt ttgatcctac caaattcgcg caaattaatg cccgtgttcc atcgggccag 16020
   cgagtttatt caaaagagtt tcgtacacgt gggcggcgac ggcaacgtca atgcttgcta 16080
   gccctaccgg cgagaagttg gccggcccct tccatgcctt gaggtcattc atcaaggcct 16140
   cgtcatcgag aatttcggtg tagttcttga tcccatcgcg cttgccgtgt tgggtcagtt 16200
   tcataccgcg cctagaatag tagagggcaa cggcatcaac gttgcgggct tccatcgcaa 16260
   caaggtcatc ggcgacaatt agaccatccg cagataggac atgctcaatg taatccggcg 16320
   gcatgtcatc aataccgagt gacaaagtga ctgcgttggg ggcgatttca gcggcttcga    16380
   ataccggttt tccgtagttg gtcgccatga tgacgaattg agaatatggc aaaaggctac    16440
   gatcgccgac agcttcaagg ctaaaggtta cgcaatcacg taacttttcg acgagctcga 16500
   aattggattt cttaccgcgg ctgagcactg ctaccttacg aattctctta gcggcaccat 16560
   agttaagtga gagaattaca gcttcggcaa cttttccagc cccaaacaag aaaacgtcga 16620
   tgtcctctct gccttgcaac agcaggttta cgcatgctag cgagaaccaa cccgttcttc 16680
   cattagaaat tgccacgccc tctaccgaca taaggagcgt cccggacacc ttgtcgcgca 16740
   ggaaaatatc ggagtgctgg agcggctttc cggtagcggc gttggttggc gcgaagtgga 16800
   tgtctttggt gccggaatat cttccgaaat agccaatgag tgctccttca gtccatccag 16860
   gaacattctt gttgaacgtt aggtaagctt tgacatgtcc ggcttttcct gcggcaaaca 16920
   cctcccaata ggacttgaga gcttcgtcaa caaatgctgg tgtgatctgg atatcgaggt 16980
   ttgatagtgc agattcagtc cagtgtacct cgcaaagttg tttggccatc tgccttgtag 17040
   gtgcgaattt tctctgctca aattgttgag gttagcggat ttgtaaacgc gtttatatgg 17100
   gctgcttgga gggtactttt ggattaattt ttttctgcca gcgcattctg acgcggcacc 17160
   gctttggaaa gtgcgctgtg ggtccgcgtt ttctacaata atgtgccgat ccggtcagaa 17220
   agtatatgga tgagttgtgc cagcctcacc aacgtgctgc aggcccatca tgactacttc 17280
   aatgttaatg ggggtaatga ataaataggc gaaattgggt tcacggtggg cccagggaat    17340
   ataatattgc cgcagaggta gtcggatgcc aaggcccgca actaatagtt cacgaacaaa 17400
   ttcattgtag tgggcggcca actccaaaac caattgccag ttattgtatt gcaatacata 17460
   tatgagtatt cggatacaac taatttcatt aaataatatt ttaagtgtgg acagaatagc 17520
   gcctaataaa tttgcgaatg ttgtccaatt gacgttttta taggtaactc gataaatcgt 17580
   gcttttgtga tattctgatg cggacaatat acatttaaac ataaagatat aagttattga 17640
   ggcatttatg tatattacaa tagtggggta catttttcac agatgctgtc acccatgaaa 17700
   tattggcaaa atactcttaa aatatgcaag aaactaaaga ggatgcatgg gttgggctgt 17760
   aggtacatgg atgcaaatgc tgttttgcaa taagtcatat agtctcgtct gttgagtgag 17820
   gcccattcaa tcagcaagta ggactgaggt gcatgatcga catatttttg aaccacagtt 17880
   ttggcaagtt tttcatacaa atgcacggct acggccaaat cgtagcttgc aagtccaact 17940
   gctgaaaagt tagccggccc gttccaagaa attagccttt gcataaggac tggatcgcgg 18000
   agaacttcag agtagttcct gatcccattg tccctgccgt gttttgttag ctttaaatgg 18060
   cgtcttgaat agtgcagcgc caacgagtcg atattacgtg tttccatcgc atccatatca 18120
   tctgccacca cgatgccact cagcttcaac acgtgatcaa aatagtcagc tggcaattcg 18180
   tcaattccaa gcgtcaatgt aacggcattg tctgtgatct ccttcatctc aaagacgggc 18240
   ttgtttgaat tcgtcgccgt aattatgaac ttggatttgc tgagatatgc tcgattgtta 18300
   acagccttga gtgaaatctt gacttccggc tgaagccttt gcaccaactc atggtttgac    18360
   tggttgcagc ggctgagaat cgcgattcgt tgaattcttc cagatgctcc cgaattgagg    18420
   gcgaggatga tggcctcggc aactttacct gctccgaata ggaagacatt gatctggctt    18480
   cggccctgca ataggagatt caggcatgct agtgccagcc aaccagttct cctctccgat    18540
   atagccaccc catcaacaga gaagagacgt ctacctgtga aacgattgcg aagccaacgt    18600
   cgatgtgaga agtcggttct ttgtatctcg cgtttgacgg attagaatgg atgcttttca    18660
   cacccgaata gtcgccgacg aaacccacca gagctccctc cgtacagccc tctcgatcaa    18720
   gtggaacgaa gaccttgttg tggccgagcc gcccttcagc aaagaggtgc caataatctt    18780
   tcaaggcatc cgcgacgagt tccggtgtaa tgtatattcc aaaagccgat agagattcct    18840
   ctgtccaaca ttgctcgtgt atttgatcgg ccatgtttgt gtttgatcag cctcctttcg    18900
   aaaatttctt gagtttcgaa taattctaaa atcgaaggac gattaatagt gccataccaa    18960
   gacaagaagg gtaggtgggc catcaatcca caagcctagc acattttgct gtctgctcat    19020
   gcaaggtatc caatggaagc ctggattggt tagccgaact tggtgggttc aattggagcg    19080
   ggcaggtcac tttttgtctc tcaaataact gaaactaagt tttgttattt ggtatgtgtt    19140
   tgtctgttct gccgaaggtg cccgaatttg cgcaaattcc tttctaaaaa ggcttacatc    19200
   tagcaaaagg tgagccctgt gcatcccagc atttggacaa agcgcgccaa ttcggacagc    19260
   gactggctgc gttggaggct cggatctcaa agaatagaaa agagttatga tcatgttcag    19320
   aaccgccaat tttgtgcggt atgagctctt tgatgaaagt aatggtttca aaaaagcaac    19380
   atcgtgggtg aaaggtacct acatatcttc acagacaata actactgttg ctgtttgctg    19440
   attgactgac aggatatatg ttcctgtcat g                                   19471
<210> 2
   <211> 5995
   <212> DNA
   <213> Agrobacterium rhizogenes
<400> 2
   gtcgacagtc gcaacagcaa tcgaggggtg ttgatcaacc ttggccagtt gcccttcgtc      60
   cctatcagta acgacaacct tgtaatcgcc ggtttccgca agcatcagag caatggctcc     120
   gccaatctta cccgcgccga taaccactat ttctttcata caaatcccct gtgttctgta     180
   tataggatat ttataaaaat aaccggggat tggtcgaatc atagcggcaa ggtgggcatt     240
   atgttatata tttttgtgca aaaagacgac tgcacttggt aatatgaagg ggttccgcat     300
   gcaattgacc gaaagggatc gggagctcct gtcgttactc ggcgaaaatg ccagaacgcc     360
   tgtggcgacg ctcgcgaaaa agctttctct atccaggacc acagtgcagg ctcgcttgga     420
   gcgccttgag agagagggag ttattgtcgg ctatggtgta aggctatcga atgaatattc     480
   ctcgagcttg gttcgagccc atattctgat caccattacg aaggcgcttt cacaggtgac      540
   ggcctccctt gggaaggtca cggccgttat cgctcttcat tctgtgagtg gcacttttga      600
   tttgattgcg attatcgaag ccccttcgat ttcagagctc gatcagttca tcgatcaaat      660
   cggcataatt gaaggtgtcg agcggacgct ctcttcgatt attctttcaa cacgcatttc      720
   acgctgaaag agcaagaggc cacagccgtg cgcctgctgc cacctttccg acgatgacca      780
   tgcagaactt acggcctcta tgtcggtgta ttccacccac cgacgcgcca tttccgtgtg      840
   cattaagata atgctttccg ccccatcgga ttgctgttct gacatggtat atcattacga      900
   cagccaatgt ttggcggctc gtacgaatca tcaagcggtc aacctgtgct gtcaaccgtc      960
   acgatatgag caagcaactc aaacgctttc ctattcaccg cacgcgcgtt gttggctgtt     1020
   tggcaggata tatgccaacg taaaaatgag ggcaatcgat tgtactgaat cggattttca     1080
   agggtctggc caaaactatt ccgtgggcac ctggcacacg ccctggagtc cggcccgttt     1140
   ccagttgagg gttgtctacg cttagatgag aaggaaagtt gtccaagacg aatcccagtg     1200
   tcctattacc aatagccggc gctagtttga tttcagaata aagagaaatt cgtcacacca     1260
   aatattagaa gcaatgtttg attgaccatc atacttaaga tgaacattcc aaacacagtt     1320
   ataatacgca ttattattga cacaatataa aattataatg ttgatattcc ttgaaatata     1380
   atattacaat agataaagta gaggaaatta tgtcagaaat ttggtttggc tctgcttacg     1440
   acagagtcgg acgatgccta aagtctattg cttcttctat cgctagtcca attgctaaca     1500
   gacgttcatc cgagcttgca gagccatcga tttccatgcc aataggcaga ccgttggaac     1560
   taagagaaac gggaagactt aggcccggca ggcccgcatt actgctggga tctacattcc     1620
   gcacgaagat tttaaaggta tcggtcattg agccattgtg aatcaccgat agatcatggc     1680
   caattggctt ggctgtcaac ggagctgttg ggaaaagaat tgcatctagc tgatgcgcct     1740
   tgaagtaact gtggtaggcc gcttggagtc tcggtctgaa aaaacgtcgc gccagacaat     1800
   actcgctttt ggaaataaga ttatccgaga gttgtgcatt gagaatactt gcaacatccg     1860
   gactgcgaat cgctctgaca acctcagaaa aggaaacacc ctctacgaag ttctgaatat     1920
   aatgttcaag ggacaacgga aattcgtaga tggcagtcgg aaagctgacc ccttcattgt     1980
   gatgcgctaa atcaggaata tctgcttcaa caaaagtaac atctttgcgt gccagaactc     2040
   tgataatcgt ctcggctgct aaggcgacat cgggctccag gtcgttgtaa aagtaagcgg     2100
   ttggcaagcc tatacgcagc cccttcaggc ggaccgtttg attaaccggc ggtctcccgc     2160
   aaatgatacc gtcaagaaga atcacgtccg gaacattctg tgcgataacg ccaggggtgt     2220
   cccgggtggg gcttaccgga actattccgt ccgttggata tcgccccacg gtaggacgaa     2280
   accccaccac gccgcacaag gcggccggta aacggaccga cgctcccgtg tcagttccga     2340
   cgccgcccag catcaatcgg ccggccaccg cggcggccac acccccactt gatccccctg 2400
   ggatgagact agggttccac gggtttcgta cggcgcctgt ggcgaagttg ttgctcgtga 2460
   tcccaaaaga caattcgtgc atgtttcccg aagcgccagg cagtgcccca gccgcgagaa 2520
   gttgtcgtgc aactccggca ggcgtcttgg gtttgtggtt ctgtaagcct ggcgtaccag     2580
   cggtcgcggc gaacctgcct gtcgcaatat tcgctttaaa gcatagggga acgccagcta     2640
   ggccaacacc ggcacctccg tgttgatcga ttttgctggc agtccaccgt aggtgcgccc 2700
   agtcggtttc cagaaaggcg tttaaggatc ttgctgcttc acagcgggct attatcgttt 2760
   cgattaactc aaagcacgag tattttcttt ccctgagaca tttaagcgtc tcggtgatcg 2820
   aggagagggt caccattttc gttgtgctga gggaactgag atagatctcg ccagagaaac 2880
   gttcaatgat ttttgcttgg agtgaaaaag gcaaataatt atagaggaag gaagtcagaa 2940
   atgctgcgca gtagggccac ttgtataagt gccggtcgaa cactgctggt ggaaagtcaa 3000
   aagcgtgaag tattagttga actctgttac taaattgaga taaatgggat attttattcg 3060
   aaagtactgt ttgagatcta gcgacaataa taatgtcatc ttatgagatt gcatggcaat 3120
   atggatctaa tatttggcat aaatagatgg tggttttgtc tccactttta aaccttcaca 3180
   gcgttaccct aacacctctt aattgcgtac actcctttca accgcatcaa tggctggatc 3240
   ctccttcaca ttgccatcaa ctggctcagc gccccttgat atgatgctta tcgatgattc 3300
   agatctgctg caattgggtc tccagcaggt attctcgaag cggtacacag agacaccgca 3360
   gtcacgctac aaactgacca ggagggcttc tccagacgtc tcatctggcg aaggcaatgt 3420
   gcatgccctt gcgttcatat atgtcaacgc tgagacgttg cagatgatca aaaacgctcg 3480
   atcgctaacc gaagcgaacg gcgtcaaaga tcttgtcgcc atcgacgttc cgccatttcg 3540
   aaacgacttc tcaagagcgc tactccttca agtgatcaac ttgttgggaa acaaccgaaa 3600
   tgccgatgac gatcttagtc acttcatagc agttgctctc ccaaacagcg cccgctctaa 3660
   gatcctaacc acggcaccgt tcgaaggaag cttgtcagaa aacttcaggg ggttcccgat 3720
   cactcgtgaa ggaaatgtgg catgtgaagt gctagcctat gggaataact tgatgcccaa 3780
   ggcctgctcc gattcctttc caaccgtgga tcttctttat gactatggca agttcttcga 3840
   gagttgcgcg gccgatggac gtatcggtta ttttcctgaa ggcgttacga aacctaaagt 3900
   ggctataatt ggcgcaggct tttccgggct cgttgcagcg agcgaactac ttcatgcagg 3960
   ggtagacgat gttacggtgt atgaggcgag tgatcggctt ggaggaaagc tatggtcaca 4020
   cggatttaag agtgctccaa atgtgatagc cgagatgggg gccatgcgtt ttccgcgaag 4080
   tgaatcatgc ttgttcttct atctcaaaaa gcacggactg gactccgttg gtctgttccc 4140
   gaatccggga agtgtcgata ccgcattgtt ctacaggggc cgtcaatata tctggaaagc 4200
   gggagaggag ccaccggagc tgtttcgtcg tgtgcaccat ggatggcgcg catttttgca 4260
   agatggctat ctccatgatg gagtcatgtt ggcgtcaccg ttagcaattg ttgacgcctt 4320
   gaatttaggg catctacagc aggcgcatgg cttctggcaa tcttggctca catattttga 4380
   gcgagagtct ttctcttctg gcatcgaaaa aatgttcttg ggcaatcatc ctccgggggg 4440
   tgaacaatgg aattccctag atgacttgga tcttttcaaa gcgctgggta ttggatccgg 4500
   cggattcggc cctgtatttg aaagtgggtt tatcgagatc cttcgcttag tcgtcaacgg 4560
   gtatgaggat aacgtgcggc tgagttacga aggaatttct gagctgcctc ataggatcgc 4620
   ctcacaggta attaacggca gatctattcg cgagcgtaca attcacgttc aagtcgagca 4680
   gattgataga gaggaggata aaataaatat caagatcaaa ggaggaaagg ttgaggtcta 4740
   tgatcgagta ctggttacat ccgggtttgc gaacatcgaa atgcgccatc tcctgacatc 4800
   aagcaacgca ttcttccatg cagatgtaag ccatgcaata gggaacagtc atatgactgg 4860
   tgcgtcaaaa ctgttcttgc tgactaacga aaaattctgg ctacaacatc atttgccatc 4920
   gtgcatactc accaccggcg ttgcaaaggc agtttattgc ttagactatg atccgcgaga 4980
   tccaagcggc aaaggactgg tgttgataag ctatacttgg gaggatgact cacataagct 5040
   cctagccgtc cccgacaaaa gagaaaggtt cgcatcgctg cagcgcgata ttgggagggc 5100
   attcccagat tttgccaagc acctaactcc tgcagacggg aactatgatg ataatatcgt 5160
   tcaacatgat tggctgactg atccccacgc tggcggagcg tttaaactga accgcagagg 5220
   caacgacgta tattcagaaa ggcttttctt tcagcccttt gacgtaatgc atcccgcgga 5280
   cgataaggga ctttacttgg ccggttgtag ctgttccttc accggagggt gggttcatgg 5340
   tgccattcag accgcatgca acgctacgtg tgcgatcatt tatggttccg gacacctgca 5400
   agagctaatc cactggcgac acctcaaaga aggtaatcca ctggcgcacg cttggaagcg 5460
   gtataggtat caagcgtgat aatgcaacag ttagaataat tagtttgccc tagccggtat 5520
   tccttggtgt tccaataggg ttccgaagcc aataggcgaa aaagctgact tttcagtccc 5580
   ttttattatt caattcgctt cggtccaagc ataattgtaa cgctacgtga tagaaaaatg 5640
   gaaattgaca gattacttac ttaaattaat ataatctatt aatatcgtca agctaaaaac 5700
   atgtaatacg taaatatatg gaaactttat gtctgaaaag accacattat tattgatcgt 5760
   aatacactga actggtcata acagggaagg ctaactgcaa catatcctat aaatactcag 5820
   tgaaaatggc cgctccccaa tgttaagcca tttttgcggt cgggctaagc gctcgtccgt 5880
   gtctcccctg gcccgagtgt cggctctcca tcagcggcct catcatctgt cgctgacacc 5940
   ggtggcccca atttcaaatc gaggaaagac gatgccctcg ccggcaaacg tcgac 5995
<210> 3
   <211> 303
   <212> DNA
   <213> Agrobacterium rhizogenes
<400> 3
   atggaattag ccggactaaa cgtcgccggc atggcccaga ccttcggagt attatcgctc       60
   gtctgttcta agcttgttag gcgtgcaaag gccaagagga aggccaaacg ggtatccccg      120
   ggcgaacgcg accatcttgc tgagccagcc aatctgagca ccactccttt ggccatgact      180
   tcccaagccc gaccgggacg ttcaacgacc cgcgagttgc tgcgaaggga ccctttgtcg      240
   ccggacgtga aaattcagac ctacgggatt aatacgcatt tcgaaacaaa cctacgggat      300
   taa                                                                    303
<210> 4
   <211> 780
   <212> DNA
   <213> Agrobacterium rhizogenes
<400> 4
   atggatccca aattgctatt ccttccacga tttcaaccag tagatctcac tccagcatgg       60
   agccagataa acctattcga ggggatccga tttgcttttg caatctatag ccgtgactat      120
   agcaaacccc tcctgcattt ccagaaacga tgggctcttg cagtgctaga tttgaaggaa      180
   aactctccac cgatatatat acttaaacaa ctagctgagc tcttgaagaa caaagtctgc      240
   tatcatcctc ctatgtttgt tagtcagccg gatctggctc gagaaaacga ccaacatgta      300
   tttgtctatc tttctcgcga gaagatgcag aaagtgctga aggaacaatc cattacattt      360
   ggaatggagg ccgtgctggc gacaacgatt caaccatatc ggagcgagct cgccctccag      420
   gagatgctcc gtgttcacaa ccttgcttgg ccgcacagcc gcacggagga acctgattta      480
   gaatgcttca tcgccatttt cgcaagttcc ttgttcattc acttgctgga gttaaaagtg      540
   accaacgttt acgggagaga ggtagcttgc accttctttc tgcggcgagg gactgaaaac      600
   cgcccctatg atgttgtagc ttgcggcacc acacaattca ccaaaaatgc cctcgggata      660
   tcacgtccgg ccgcctcctc accggagcca gacctaaccc tgcgactctc ggggcctgat      720
   caggaaggcg aggagggcgt catgaagcct gctgcagtaa acctgaagaa agaagcctaa      780
<210> 5
   <211> 543
   <212> DNA
   <213> Agrobacterium rhizogenes
<400> 5
   atggctgaag acgacctgtg ttctctcttt ttcaagctca aagtggagga tgtgacaagc        60
   agcgatgagc tagctagaca catgaagaac gcctcaaatg agcgtaaacc cttgatcgag       120
   ccgggtgaga atcaatcgat ggatattgac gaagaaggag ggtcggtggg ccacgggctg       180
   ctgtacctct acgtcgactg cccgacgatg atgctctgct tctatggagg gtccttgcct       240
   tacaattgga tgcaaggcgc actcctcacc aaccttcccc cgtaccagca tgatgtgact       300
   ctcgatgagg tcaatagagg gctcaggcaa gcatcaggtt ttttcggtta cgcggatcct       360
   atgcggagcg cctacttcgc tgcattttct ttccctgggc gtgtcatcaa gctgaatgag       420
   cagatggagc taacttcgac aaagggaaag tgtctgacat tcgacctcta tgccagcacc       480
   cagcttaggt tcgaacctgg tgagttggtg aggcatggcg agtgcaagtt tgcaatcggc       540
   taa                                                                     543
<210> 6
   <211> 603
   <212> DNA
   <213> Agrobacterium rhizogenes
<400> 6
   atggctcgtt attgcagtgg tggctctcaa cggcttcatg tcgatgattt tcgttggatc        60
   aaggagccca ctcgactgaa ggctcagctt attaatgtgg tggagaccta caaggctgca       120
   caaacagaga cgttaaagta ctatatatca tctgcaactg agcgtgtggc tcatgtggag       180
   gcagccgagg tcaacaatgc ggaaatggag ctgcatcctg ctgggttgaa gtaccctctg       240
   tccttcgtct ttacctccct ggccgtggct acagcctgca aggagaacaa gcatctcttg       300
   tgcgaggagc atttggaggg ggacttgata tcgtgcgtcg ttcctcccta tcagacaaat       360
   gtctcactcg ctgctttaag ggagctccac aattccattt cgggaggagg gtaccaggaa       420
   caagcagaca tggattattt tgtggcgatc atcccaaatg ataatttcga ctatcagagc       480
   tgcgaaatcg acacacgaag ttgcggtaaa ggactttgca agatttatag tagggaactg       540
   ggagggcagc ctctagctta tgacgccata ctggcaatcg gcaaggtgct gctgctggaa       600
   tag                                                                     603
<210> 7
   <211> 2250
   <212> DNA
   <213> Agrobacterium rhizogenes
<400> 7
   atggctggat cctccttcac attgccatca actggctcag cgccccttga tatgatgctt      60
   atcgatgatt cagatctgct gcaattgggt ctccagcagg tattctcgaa gcggtacaca     120
   gagacaccgc agtcacgcta caaactgacc aggagggctt ctccagacgt ctcatctggc     180
   gaaggcaatg tgcatgccct tgcgttcata tatgtcaacg ctgagacgtt gcagatgatc     240
   aaaaacgctc gatcgctaac cgaagcgaac ggcgtcaaag atcttgtcgc catcgacgtt     300
   ccgccatttc gaaacgactt ctcaagagcg ctactccttc aagtgatcaa cttgttggga     360
   aacaaccgaa atgccgatga cgatcttagt cacttcatag cagttgctct cccaaacagc     420
   gcccgctcta agatcctaac cacggcaccg ttcgaaggaa gcttgtcaga aaacttcagg     480
   gggttcccga tcactcgtga aggaaatgtg gcatgtgaag tgctagccta tgggaataac      540
   ttgatgccca aggcctgctc cgattccttt ccaaccgtgg atcttcttta tgactatggc      600
   aagttcttcg agagttgcgc ggccgatgga cgtatcggtt attttcctga aggcgttacg      660
   aaacctaaag tggctataat tggcgcaggc ttttccgggc tcgttgcagc gagcgaacta      720
   cttcatgcag gggtagacga tgttacggtg tatgaggcga gtgatcggct tggaggaaag      780
   ctatggtcac acggatttaa gagtgctcca aatgtgatag ccgagatggg ggccatgcgt      840
   tttccgcgaa gtgaatcatg cttgttcttc tatctcaaaa agcacggact ggactccgtt      900
   ggtctgttcc cgaatccggg aagtgtcgat accgcattgt tctacagggg ccgtcaatat      960
   atctggaaag cgggagagga gccaccggag ctgtttcgtc gtgtgcacca tggatggcgc     1020
   gcatttttgc aagatggcta tctccatgat ggagtcatgt tggcgtcacc gttagcaatt     1080
   gttgacgcct tgaatttagg gcatctacag caggcgcatg gcttctggca atcttggctc     1140
   acatattttg agcgagagtc tttctcttct ggcatcgaaa aaatgttctt gggcaatcat     1200
   cctccggggg gtgaacaatg gaattcccta gatgacttgg atcttttcaa agcgctgggt     1260
   attggatccg gcggattcgg ccctgtattt gaaagtgggt ttatcgagat ccttcgctta     1320
   gtcgtcaacg ggtatgagga taacgtgcgg ctgagttacg aaggaatttc tgagctgcct     1380
   cataggatcg cctcacaggt aattaacggc agatctattc gcgagcgtac aattcacgtt     1440
   caagtcgagc agattgatag agaggaggat aaaataaata tcaagatcaa aggaggaaag     1500
   gttgaggtct atgatcgagt actggttaca tccgggtttg cgaacatcga aatgcgccat     1560
   ctcctgacat caagcaacgc attcttccat gcagatgtaa gccatgcaat agggaacagt     1620
   catatgactg gtgcgtcaaa actgttcttg ctgactaacg aaaaattctg gctacaacat     1680
   catttgccat cgtgcatact caccaccggc gttgcaaagg cagtttattg cttagactat     1740
   gatccgcgag atccaagcgg caaaggactg gtgttgataa gctatacttg ggaggatgac     1800
   tcacataagc tcctagccgt ccccgacaaa agagaaaggt tcgcatcgct gcagcgcgat     1860
   attgggaggg cattcccaga ttttgccaag cacctaactc ctgcagacgg gaactatgat     1920
   gataatatcg ttcaacatga ttggctgact gatccccacg ctggcggagc gtttaaactg     1980
   aaccgcagag gcaacgacgt atattcagaa aggcttttct ttcagccctt tgacgtaatg     2040
   catcccgcgg acgataaggg actttacttg gccggttgta gctgttcctt caccggaggg     2100
   tgggttcatg gtgccattca gaccgcatgc aacgctacgt gtgcgatcat ttatggttcc     2160
   ggacacctgc aagagctaat ccactggcga cacctcaaag aaggtaatcc actggcgcac     2220
   gcttggaagc ggtataggta tcaagcgtga                                      2250
<210> 8
   <211> 1401
   <212> DNA
   <213> Agrobacterium rhizogenes
<400> 8
   atggtgaccc tctcctcgat caccgagacg cttaaatgtc tcagggaaag aaaatactcg      60
   tgctttgagt taatcgaaac gataatagcc cgctgtgaag cagcaagatc cttaaacgcc     120
   tttctggaaa ccgactgggc gcacctacgg tggactgcca gcaaaatcga tcaacacgga     180
   ggtgccggtg ttggcctagc tggcgttccc ctatgcttta aagcgaatat tgcgacaggc     240
   aggttcgccg cgaccgctgg tacgccaggc ttacagaacc acaaacccaa gacgcctgcc     300
   ggagttgcac gacaacttct cgcggctggg gcactgcctg gcgcttcggg aaacatgcac     360
   gaattgtctt ttgggatcac gagcaacaac ttcgccacag gcgccgtacg aaacccgtgg     420
   aaccctagtc tcatcccagg gggatcaagt gggggtgtgg ccgccgcggt ggccggccga     480
   ttgatgctgg gcggcgtcgg aactgacacg ggagcgtcgg tccgtttacc ggccgccttg     540
   tgcggcgtgg tggggtttcg tcctaccgtg gggcgatatc caacggacgg aatagttccg     600
   gtaagcccca cccgggacac ccctggcgtt atcgcacaga atgttccgga cgtgattctt     660
   cttgacggta tcatttgcgg gagaccgccg gttaatcaaa cggtccgcct gaaggggctg     720
   cgtataggct tgccaaccgc ttacttttac aacgacctgg agcccgatgt cgccttagca     780
   gccgagacga ttatcagagt tctggcacgc aaagatgtta cttttgttga agcagatatt     840
   cctgatttag cgcatcacaa tgaaggggtc agctttccga ctgccatcta cgaatttccg     900
   ttgtcccttg aacattatat tcagaacttc gtagagggtg tttccttttc tgaggttgtc     960
   agagcgattc gcagtccgga tgttgcaagt attctcaatg cacaactctc ggataatctt    1020
   atttccaaaa gcgagtattg tctggcgcga cgttttttca gaccgagact ccaagcggcc    1080
   taccacagtt acttcaaggc gcatcagcta gatgcaattc ttttcccaac agctccgttg    1140
   acagccaagc caattggcca tgatctatcg gtgattcaca atggctcaat gaccgatacc    1200
   tttaaaatct tcgtgcggaa tgtagatccc agcagtaatg cgggcctgcc gggcctaagt    1260
   cttcccgttt ctcttagttc caacggtctg cctattggca tggaaatcga tggctctgca    1320
   agctcggatg aacgtctgtt agcaattgga ctagcgatag aagaagcaat agactttagg    1380
   catcgtccga ctctgtcgta a                                              1401
<210> 9
   <211> 20
   <212> DNA
   <213> Artificial Sequence
<220>
   <223> amplification primer
<400> 9
   ccaatctgag caccactcct 20
<210> 10
   <211> 20
   <212> DNA
   <213> Artificial Sequence
<220>
   <223> Amplification primer
<400> 10
   aatcccgtag gtttgtttcg 20
<210> 11
   <211> 20
   <212> DNA
   <213> Artificial Sequence
<220>
   <223> Amplification primer
<400> 11
   gatatcccga gggcattttt 20
<210> 12
   <211> 20
   <212> DNA
   <213> Artificial Sequence
<220>
   <223> Amplification primer
<400> 12
   gaatgcttca tcgccatttt 20
<210> 13
   <211> 20
   <212> DNA
   <213> Artificial Sequence
<220>
   <223> Amplification primer
<400> 13
   caatagaggg ctcaggcaag 20
<210> 14
   <211> 20
   <212> DNA
   <213> Artificial Sequence
<220>
   <223> Amplification primer
<400> 14
   cctcaccaac tcaccaggtt 20
<210> 15
   <211> 19
   <212> DNA
   <213> Artificial Sequence
<220>
   <223> Amplification primer
<400> 15
   gcgaagtgga tgtctttgg 19
<210> 16
   <211> 21
   <212> DNA
   <213> Artificial Sequence
<220>
   <223> Amplification primer
<400> 16
   ttgcgaggta cactggactg a 21
<210> 17
   <211> 20
   <212> DNA
   <213> Artificial Sequence
<220>
   <223> Amplification primer
<400> 17
   gcaggacgtg atctgactga 20
<210> 18
   <211> 20
   <212> DNA
   <213> Artificial Sequence
<220>
   <223> Amplification primer
<400> 18
   gacggacgag ctactcttgg 20